# EUROPEAN PATENT SPECIFICATION

(11) **EP 3 743 116 B1**
(45) Date of publication and mention of the grant of the patent: **10.07.2024**
(21) Application number: 19701516.7
(22) Date of filing: 24.01.2019
(51) Int. Cl.: A61K 51/04, C07H 21/00

(54) **RADIOLABELLED OLIGONUCLEOTIDES AND PROCESS FOR THEIR PREPARATION**
RADIOAKTIV MARKIERTE OLIGONUKLEOTIDE UND VERFAHREN ZU DEREN HERSTELLUNG
OLIGONUCLÉOTIDES RADIOMARQUÉS ET LEUR PROCÉDÉ DE PRÉPARATION

(30) Priority: 26.01.2018 EP 18153591
(43) Date of publication of application: 02.12.2020
(73) Proprietor: F. Hoffmann-La Roche AG, 4070 Basel (CH)
(72) Inventor: EDELMANN, Martin Robert, 4070 Basel (CH)
(74) Representative: Rauber, Beat
(86) International application number: PCT/EP2019/051682
(87) International publication number: WO 2019/145384

(56) References cited:
- KOJIMA N ET AL: "Efficient synthesis of oligonucleotide conjugates on solid-support using an (aminoethoxycarbonyl)aminohexyl group for 5'-terminal modification", BIOORGANIC & MEDICINAL CHEMISTRY LETTERS, PERGAMON, AMSTERDAM, NL, vol. 19, no. 8, 15 April 2009 (2009-04-15), pages 2144 - 2147, XP026079425, ISSN: 0960-894X, [retrieved on 20090304], DOI: 10.1016/J.BMCL.2009.02.121
- JAMES A. H. INKSTER ET AL: "Labeling of an Antisense Oligonucleotide with [ 18 F]FPy5yne", NUCLEOSIDES, NUCLEOTIDES AND NUCLEIC ACIDS., vol. 28, no. 11-12, 7 December 2009 (2009-12-07), US, pages 1131 - 1143, XP055250456, ISSN: 1525-7770, DOI: 10.1080/15257770903400691
- KOBORI N ET AL: "VISUALIZATION OF MRNA EXPRESSION IN CNS USING 11C-LABELED PHOSPHOROTHIOATE OLIGODEOXYNUCLEOTIDE", NEUROREPORT, LIPPINCOTT WILLIAMS & WILKINS, UK, vol. 10, no. 14, 29 September 1999 (1999-09-29), pages 2971 - 2974, XP009014077, ISSN: 0959-4965, DOI: 10.1097/00001756-199909290-00018
- JAYAPRAKASH K. NAIR ET AL: "Multivalent N -Acetylgalactosamine-Conjugated siRNA Localizes in Hepatocytes and Elicits Robust RNAi-Mediated Gene Silencing", JOURNAL OF THE AMERICAN CHEMICAL SOCIETY, vol. 136, no. 49, 10 December 2014 (2014-12-10), pages 16958 - 16961, XP055181463, ISSN: 0002-7863, DOI: 10.1021/ja505986a

## Description

The invention relates to novel radiolabeled oligonucleotide of the formula I 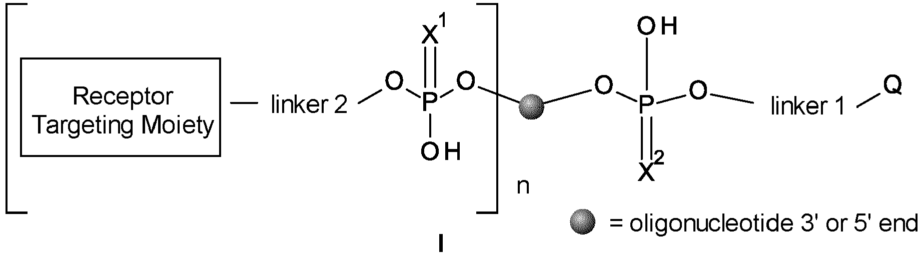 wherein,
n, X¹ and X², the linkers 1 and 2, Q and the receptor targeting moiety are discussed hereinafter, a process for their preparation and to their use for the determination of the biodistribution and pharmacokinetics of the oligonucleotide in the tissue or body fluid.

For an antisense therapeutic approach to be effective, oligonucleotides must be introduced into a patient and must reach the specific tissues to be treated. The biodistribution and pharmacokinetics of a therapeutic drug must be determined as a step preliminary to treatment with the drug. Consequently, there is a need to be able to detect oligonucleotides in body fluids or tissues. Agrawal et al., Clin. Pharmacokinetics 28, 7 (1995), reviews certain aspects of the pharmacokinetics of antisense oligonucleotides. Another well-established approach used in *in vivo* pharmacokinetic studies of pharmacological compounds such as antisense oligonucleotides entails radiolabeling the compounds to enable detection. In animal models, radiolabeled oligonucleotides have been administered to the animal and their distribution within body fluids and tissues has been assessed by extraction of the oligonucleotides followed by autoradiography (See Agrawal et al., Proc. Natl. Acad. Sci. 88, 7595-7599 (1991).

Nair J.N. et al., J. Am. Chem. Soc. 2014, 136, 16958-16961 disclose that conjugation of siRNA to an N-acetylgalactosamine (GalNAc) ligand facilitates targeted delivery of the siRNA to hepatocytes in vitro and in vivo.

³⁵S-labeling is an established and wide-spread technique. For biological studies, ³⁵S-labeled oligonucleotide phosphorothioates have been prepared using H-phosphonate chemistry (See Garegg et al., Chem. Scr. 25, 280-282 (1985).

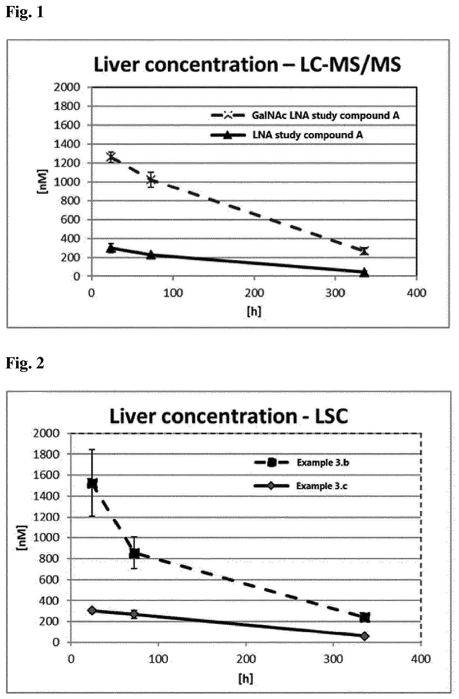

Inkster J. et al., Nucleosides, Nucleotides and Nucleic Acids, 28:1131-1143, 2009 discloses the labeling of antisense oligonucleotides with ¹⁸F and Kobori N. et al., NeuroReport 10, 2971-2974 (1999) describes the visualization of mRNA expression in CNS with ¹¹C labeled phosphorothioate oligodeoxynucleotide Radioisotopic labeling of synthetic oligonucleotides with ¹⁴C and ³H is currently accomplished by using the well-established solid-phase automated synthesis. In this approach, the assembly of ¹⁴C or ³H nucleoside phosphoramidite requires a two-step process as shown in Fig. 1 of US 5,847,104. However, several disadvantages are associated with this method. Since the radioisotope is introduced in the very first step, (a) the radiochemical yield after two steps is limited; (b) this operation often suffers a dilution problem, namely, the natural abundance isotope is usually blended in as a carrier in order to maintain a manageable synthetic scale, resulting in lower specific activity of the final oligos and (c) the phosphoramidite 3 (Fig. 1) is a reactive species prone to degradation which as the final radioactive precursor leads to stringent storage and transportation requirements.

In view of the deficiencies of the prior art methods other approaches for obtaining radiolabeled oligonucleotides with high specific activity are desirable.

Object of the invention therefore is to provide a new approach for the radiolabeling of oligonucleotides.

It was found that the objective could be fulfilled with the newly developed radiolabeled oligonucleotide of the formula I 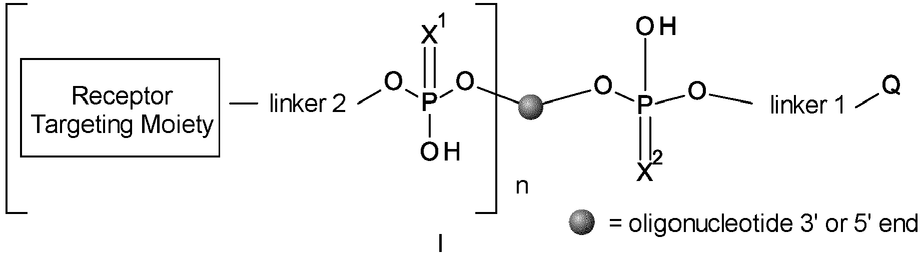 wherein,
n is 0 or 1;
X¹ and X² independently of each other are S or O;
linker 1 is a C₂₋₁₂- alkylene bridge, an ethylene glycol bridge containing 1 to 10 ethylene glycol units or a glycerol based bridge of the formula 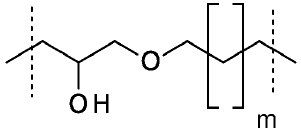
wherein m is an integer of 1 to 6;
linker 2 is an optionally amino group protected amino C₂₋₁₂-alkylene bridge, an amino ethylene glycol bridge containing 1 to 10 ethylene glycol units;
Q stands for a residue of the formula 2a or 2b 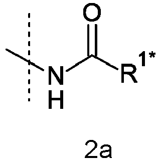 or 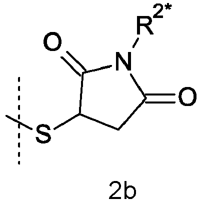
wherein R^{1*} and R^{2*} are C₁₋₆-alkyl labelled with ³H- or ¹⁴ C;
and the receptor targeting moiety is a moiety which adds additional functionality to the oligonucleotide.

The Figures have the following meaning:
In Fig. 1 the liver concentration of a GalNAc study compound A (dotted line) and a study compound A without GalNAc (continuous line) have been compared with LC-MS/MS.
In Fig. 2 the liver concentration of the tritium labeled compounds of Example 3b (dotted line) and Example 3c (continuous line) have been compared with LSC.

The following definitions are set forth to illustrate and define the meaning and scope of the various terms used to describe the invention herein.

The term "C₁₋₆-alkyl" denotes a monovalent linear or branched saturated hydrocarbon group of 1 to 6 carbon atoms, and in more particular embodiments 1 to 4 carbon atoms. Examples of C₁₋₆-alkyl include methyl, ethyl, propyl, isopropyl, n-butyl, i-butyl, sec-butyl, or *t-*butyl, preferably methyl or ethyl, more preferably ethyl.

The term "C₂₋₁₂-alkyl" likewise denotes a monovalent linear or branched saturated hydrocarbon group of 2 to 12 carbon atoms, in a more particular embodiment 4 to 8 carbon atoms and even more particular embodiment of 6 carbon atoms. Particular examples are butyl, pentyl, hexyl, heptyl or octyl and its isomers, but preferably n-hexyl.

The term "C₂₋₁₂- alkylene bridge" stands for a bivalent linear or branched saturated hydrocarbon group of 2 to 12 carbon atoms, in a more particular embodiment 4 to 8 carbon atoms and in an even more particular embodiment of 6 carbon atoms. Particular examples are butylene, pentylene, hexylene, heptylene or octylene and its isomers, but preferably n-hexylene.

The term "amino C₂₋₁₂- alkylene bridge" stands for a bivalent group comprising an amino group attached to a branched saturated hydrocarbon group of 2 to 12 carbon atoms, in a more particular embodiment 4 to 8 carbon atoms and in an even more particular embodiment of 6 carbon atoms. Particular examples are amino butylene, amino pentylene, amino hexylene, amino heptylene or amino octylene and its isomers, but preferably amino n-hexylene (-NH-(CH₂)₆-).

The term "ethylene glycol units" stands for units of the formula -(CH₂)₂-O- which as a bridging unit can contain 1 to 10 ethylene glycol units, preferably 2 to 6 ethylene glycol units.

The term "glycerol unit glycerol based bridge" is characterized by the formula 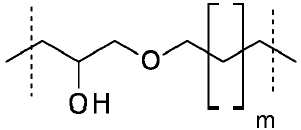 wherein m is an integer of 1 to 6, preferably 1 to 3, more preferably 1.

The term "amino-protecting group" denotes groups intended to protect an amino group and includes benzoyl, benzyloxycarbonyl, carbobenzyloxy (CBZ or Z), 9-fluorenylmethyloxycarbonyl (FMOC), p-methoxybenzyloxycarbonyl, *p-*nitrobenzyloxycarbonyl, t-butoxycarbonyl (BOC), and trifluoroacetyl. Further examples of these groups are found in T. W. Greene and P. G. M. Wuts, "Protective Groups in Organic Synthesis", 2nd ed., John Wiley & Sons, Inc., New York, NY, 1991, chapter 7; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981.

The term oligonucleotide as used herein is defined as it is generally understood by the skilled person as a molecule comprising two or more covalently linked nucleotides. For use as a therapeutically valuable oligonucleotide, oligonucleotides are typically synthesized as 7 - 30 nucleotides in length.

The oligonucleotides may consist of optionally modified DNA, RNA or LNA nucleoside monomers or combinations thereof.

The LNA nucleoside monomers are modified nucleosides which comprise a linker group (referred to as a biradical or a bridge) between C2' and C4' of the ribose sugar ring of a nucleotide. These nucleosides are also termed bridged nucleic acid or bicyclic nucleic acid (BNA) in the literature.

Optionally modified as used herein refers to nucleosides modified as compared to the equivalent DNA, RNA or LNA nucleoside by the introduction of one or more modifications of the sugar moiety or the nucleo base moiety. In a preferred embodiment the modified nucleoside comprises a modified sugar moiety, and may for example comprise one or more 2' substituted nucleosides and/or one or more LNA nucleosides. The term modified nucleoside may also be used herein interchangeably with the term "nucleoside analogue" or modified "units" or modified "monomers".

The DNA, RNA or LNA nucleosides are as a rule linked by a phosphodiester (P=O) and / or a phosphorothioate (P=S) internucleoside linkage which covalently couples two nucleosides together.

Accordingly, in some oligonucleotides all internucleoside linkages may consist of a phosphodiester (P=O), in other oligonucleotides all internucleoside linkages may consist of a phosphorothioate (P=S) or in still other oligonucleotides the sequence of internucleoside linkages vary and comprise both phosphodiester (P=O) and phosphorothioate (P=S) internucleoside.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are described with capital letters A, T, G and ^{Me}C (5-methyl cytosine) for LNA nucleoside and with small letters a,t,g,c and ^{Me}c for DNA nucleosides. Modified nucleobases include but are not limited to nucleobases carrying protecting groups such as t-butylphenoxyacetyl, phenoxyacetyl, benzoyl, acetyl, i-butyryl or dimethylformamidino (see Wikipedia, Phosphoramidit-Synthese, https://de.wikipedia.org/wiki/Phosphoramidit-Synthese of March 24, 2016).

Preferably the oligonucleotide consists of optionally modified DNA or LNA nucleoside monomers or combinations thereof and is 10 to 25 nucleotides in length.

The principles of the oligonucleotide synthesis are well known in the art and well described in literature and public for example Wikipedia (see e.g. Oligonucleotide synthesis; Wikipedia, the free encyclopedia; https://en.wikipedia.org/wiki/Oligonucleotide_synthesis, of March 15, 2016).

Larger scale oligonucleotide synthesis nowadays is carried automatically using computer controlled synthesizers.

As a rule, oligonucleotide synthesis is a solid-phase synthesis, wherein the oligonucleotide being assembled is covalently bound, *via* its 3'-terminal hydroxy group, to a solid support material and remains attached to it over the entire course of the chain assembly. Suitable supports are the commercial available macroporous polystyrene supports like the Primer support 5G from GE Healthcare or the NittoPhase^{®}HL support from Kinovate.

The oligonucleotide synthesis in principle is a stepwise addition of nucleotide residues to the 5'-terminus of the growing chain until the desired sequence is assembled.

As a rule, each addition is referred to as a synthetic cycle and in principle consists of the chemical reactions
a₁) de-blocking the protected hydroxyl group on the solid support,
a₂) coupling the first nucleoside as activated phosphoramidite with the free hydroxyl group on the solid support,
as) oxidizing or sulfurizing the respective P-linked nucleoside to form the respective phosphotriester (P=O) or the respective phosphorothioate (P=S);
a₄) optionally, capping any unreacted hydroxyl groups on the solid support;
a₅) de-blocking the 5' hydroxyl group of the first nucleoside attached to the solid support;
a₆) coupling the second nucleoside as activated phosphoramidite to form the respective P-linked dimer;
a₇) oxidizing or sulfurizing the respective P-linked dinucleoside to form the respective phosphotriester (P=O) or the respective phosphorothioate (P=S);
a₈) optionally, capping any unreacted 5' hydroxyl groups;
a₉) repeating the previous steps as to as until the desired sequence is assembled.

The term "radiolabeled" in the context of the present invention is used for the substituents R^{1*} and R^{2*} which are C₁₋₆-alkyl groups labelled with ³H or ¹⁴C, preferably a C₁₋₄-alkyl group labelled with ³H or ¹⁴ C , more preferably a methyl or ethyl group labelled with ³H or ¹⁴ C. More preferred radiolabeling is ³H.

The term "receptor targeting moiety" stands for a moiety which adds additional functionality to the oligonucleotide.

Such moieties can be selected from any protein receptor target moiety which has the potential to enhance functionality to the oligonucleotide. They include, but are not limited to antibodies or functional peptides or oligonucleotides which target specific molecules like aptamers or non-nucleotide protein receptor target moieties which have the potential to enhance delivery of the oligonucleotide to body tissue or body fluid.

In a preferred embodiment the receptor targeting moiety is an asialglycoprotein receptor targeting moiety, more preferably a GalNAc moiety.

The GalNAc moiety has the formula VII 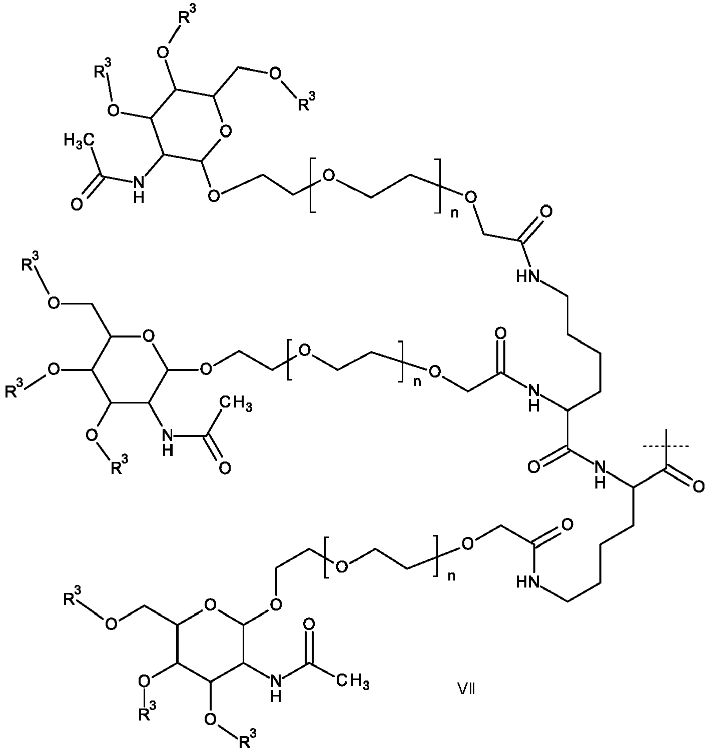 wherein R³ is hydrogen or a hydroxy protecting group and n is an integer from 0 to 10, preferably from 0 to 5, more preferably from 1 to 3, but most preferred is 2, corresponding salts, enantiomers and/ or a stereoisomer thereof.

Suitable hydroxy protecting groups are acyl, particularly the C₁₋₁₂-alkylcarbonyl group, more particularly the C₁₋₆-alkylcarbonyl group which is optionally substituted by C₁₋₆-alkyl or phenyl. More preferred is acetyl, pivaloyl or benzoyl, whereby acetyl is the most preferred hydroxy protecting group.

In a preferred embodiment the GalNAc moiety has the formula VII wherein R³ is hydrogen and n is 2.

The GalNAc moiety is connected with linker 2 *via* a peptide bond -CO-NH-.

The GalNAc cluster compounds can be prepared according to the PCT Publication WO2017021385.

In a preferred embodiment the radiolabeled oligonucleotide of formula 1 Q has the formula 2b and the conjugation is at the 3' or 5' end of the oligonucleotide.

In another preferred embodiment radiolabeled oligonucleotide of claim 1or 2, wherein Q has the formula 2a and the conjugation is at the 3' or 5' end of the oligonucleotide.

Particularly preferred are radiolabeled oligonucleotides of formula 1 wherein Q has the formula 2b and the conjugation is at the 3' or 5' end of the oligonucleotide.

In another embodiment the radiolabeled oligonucleotide has the formula Ib 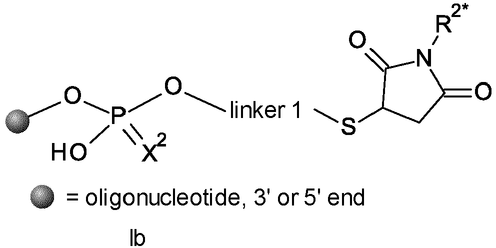 wherein
R^{2*} is C₁₋₆-alkyl labelled with ³H- or ¹⁴C;
X² is S or O;
linker 1 is a C₂₋₁₂- alkylene bridge, an ethylene glycol bridge containing 1 to 10 ethylene glycol units or a glycerol based bridge of the formula 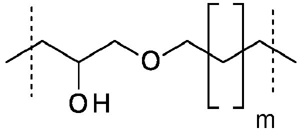
wherein m is an integer of 1 to 6.

In a preferred embodiment the radiolabeled oligonucleotide of the formula Ib has a conjugation at the 3' end.

In another preferred embodiment of the radiolabeled oligonucleotide of the formula Ib R^{2*}is methyl or ethyl, more preferably ethyl.

In another preferred embodiment of the radiolabeled oligonucleotide of the formula Ib X² is S.

In another preferred embodiment of the radiolabeled oligonucleotide of the formula Ib the linker 1 is a C₂₋₁₂- alkylene bridge, preferably a C₆- alkylene bridge.

Even more preferred is the radiolabeled oligonucleotide of the formula Ib, wherein R^{2*}is methyl or ethyl, preferably ethyl; X² is S and the linker 1 is a C₆- alkylene bridge.

In another embodiment the radiolabeled oligonucleotide has the formula Ic 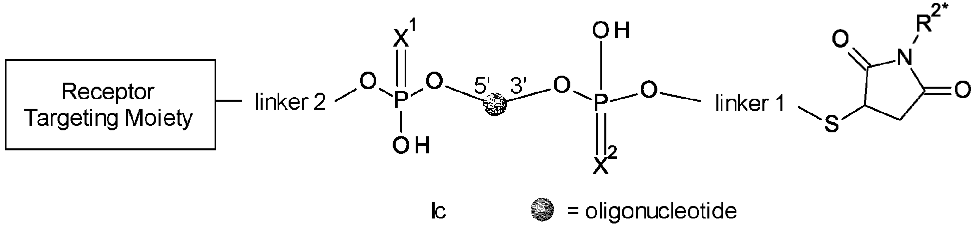 wherein,
R^{2*} is C₁₋₆-alkyl labelled with ³H- or ¹⁴C;
X¹ and X² independently of each other are S or O;
linker 1 is a C₂₋₁₂- alkylene bridge, an ethylene glycol bridge containing 1 to 10 ethylene glycol units or a glycerol based bridge of the formula 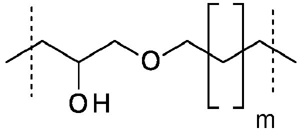
wherein m is an integer of 1 to 6;
linker 2 is an optionally amino group protected amino C₂₋₁₂-alkylene bridge, an amino ethylene glycol bridge containing 1 to 10 ethylene glycol units;
and the receptor targeting moiety is a moiety which adds additional functionality to the oligonucleotide.

The receptor targeting moiety is as defined above, but preferably an asialglycoprotein receptor targeting moiety, more preferably a GalNAc moiety.

In a preferred embodiment of the radiolabeled oligonucleotide of the formula Ic, R^{2*}is methyl or ethyl, more preferably ethyl.

In another preferred embodiment of the radiolabeled oligonucleotide of the formula Ic, X¹ is O and X² is S.

In another preferred embodiment of the radiolabeled oligonucleotide of the formula Ic the linker 1 is a C₂₋₁₂- alkylene bridge, preferably a C₆- alkylene bridge.

In another preferred embodiment of the radiolabeled oligonucleotide of the formula Ic the linker 2 is an amino C₂₋₁₂-alkylene bridge, preferably an amino C₆ - alkylene bridge.

In another preferred embodiment of the radiolabeled oligonucleotide of the formula Ic the receptor targeting moiety is a GalNAc moiety of formula V
Even more preferred is the radiolabeled oligonucleotide of the formula Ic, wherein R^{2*}is methyl or ethyl, preferably ethyl; X¹ is O and X² is S; the linker 1 is a C₆ - alkylene bridge; the linker 2 is an amino C₆ - alkylene bridge and the receptor targeting moiety is a GalNAc moiety of formula V with R³ hydrogen and n=2.

In another embodiment the radiolabeled oligonucleotide has the formulae Id 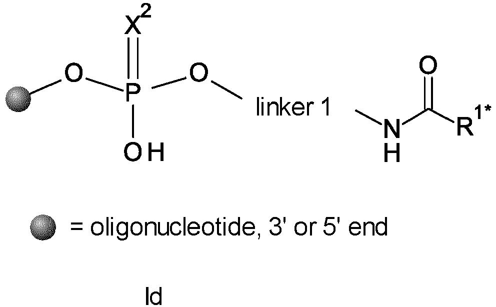 wherein
R^{1*} is C₁₋₆-alkyl labelled with ³H- or ¹⁴C;
X² is S or O;
linker 1 is a C₂₋₁₂- alkylene bridge, an ethylene glycol bridge containing 1 to 10 ethylene glycol units or a glycerol based bridge of the formula 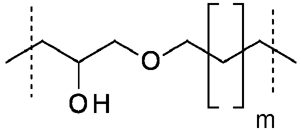
wherein m is an integer of 1 to 6.

In a preferred embodiment of the radiolabeled oligonucleotide of the formula Id, R^{1*} is methyl or ethyl, more preferably ethyl.

In another preferred embodiment of the radiolabeled oligonucleotide of the formula Id, X² is S.

In another preferred embodiment of the radiolabeled oligonucleotide of the formula Ic the linker 1 is a C₂₋₁₂- alkylene bridge, preferably a C₆ - alkylene bridge.

Even more preferred is the radiolabeled oligonucleotide of the formula Id, wherein R^{1*} is methyl or ethyl, preferably ethyl; X² is S and the linker 1 is a C₆ - alkylene bridge.

The radiolabeled oligonucleotide of the formula Id can be illustrated with the following compounds.
Prop-5'-Am-C6*G*C*a*t*t*g*g*t*a*t*T*C*A
G*A*G*t*t*a*c*t*t*g*c*c*a*A*C*T*-Am-GBB-Prop
G*C*a*t*t*g*g*t*a*t*T*C*A*-Am-GBB-Prop
wherein Am-C6 means a C6 (hexylene) amino linker; Am-GBB means a glycerol based bridge (m = 1) amino linker, Prop is a ³H labeled propionyl; * stands for phosphorthioate bridges; A,C,G,T are LNA nucleoside monomers and a,t,c,g are DNA nucleoside monomers.

Most preferred embodiments are the radiolabeled oligonucleotide of the formula Ib and Ic.

The radiolabeled oligonucleotide of the formula Ib and Ic can be illustrated with the following compounds.
5'-GN2-C6-caG*A*G*t*t*a*c*t*t*g*c*c*a*A*C*T*-C6SH-NEM
G*C*a*t*t*g*g*t*a*t*T*C*A*-C6SH-NEM
G*C*a*t*t*g*g*t*a*t*T*C*A*-C6SH-NMM
G*A*G*t*t*a*c*t*t*g*c*c*a*A*C*T*-C6SH-NEM
G*A*G*t*t*a*c*t*t*g*c*c*a*A*C*T*-C6SH-NMM
5'-NEM-SH-C6*T*T*A*c*A*c*t*t*a*a*t*t*a*t*a*c*t*T*C*C
wherein C6SH means a C6 (hexylene) thiol linker; NEM is a ³H labeled N-ethylmaleimide; NMM is a ³H labeled N-methylmaleimide; * stands for phosphorthioate bridges; A,C,G,T are LNA nucleoside monomers and a,t,c,g are DNA nucleoside monomers.

The radiolabeled oligonucleotides of the present invention have a specific activity of 37 GBq/mmol (1 Ci/mmol) to 3.7 TBq/mmol (100 Ci/mmol), preferably of 111 GBq/mmol (3 Ci/mmol) to 1.85 TBq/mmol (50 Ci/mmol), more preferably of 185 GBq/mmol (5 Ci/mmol) to 740 GBq/mmol (20 Ci/mmol).

The invention also comprises a process for the preparation of a radiolabeled oligonucleotide of the formula I.

For those radiolabeled oligonucleotides of the formula I wherein Q stands for the residue of the formula 2a the process comprises conjugating an amine of formula III 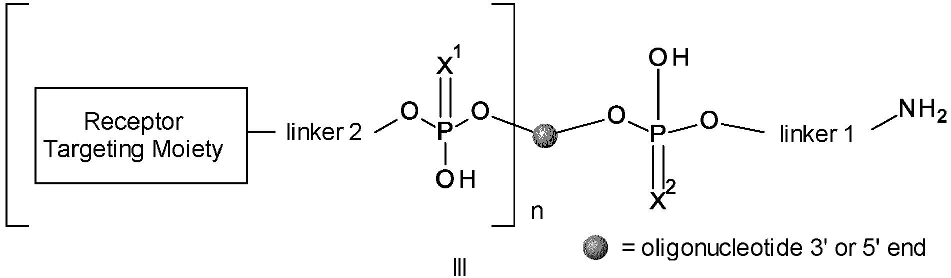 wherein,
X¹ and X² independently of each other are S or O;
linker 1 is a C₂₋₁₂- alkylene bridge, an ethylene glycol bridge containing 1 to 10 ethylene glycol units or a glycerol based bridge of the formula 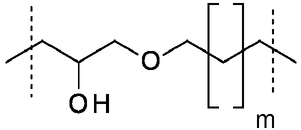
wherein m is an integer of 1 to 6;
linker 2 is an optionally amino group protected amino C₂₋₁₂-alkylene bridge, an amino ethylene glycol bridge containing 1 to 10 ethylene glycol units;
and the receptor targeting moiety is a moiety which adds additional functionality to the oligonucleotide;
with a radiolabeled succinimidyl compound of formula IV 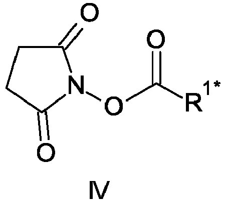
wherein R^{1*} is as above.

Radiolabeled succinimidyl derivatives are commercially available. The ³H labeled succinimidyl compound of formula IV with R^{1*} ethyl (N-succinimidyl propionate; NSP) can for instance be obtained from Pharmaron, Cardiff, UK.

The conjugation reaction can be performed in the presence of an organic base and an organic solvent or in an aqueous buffered system at a reaction temperature of 0°C to 50°C.

Suitable organic bases are tertiary amines such as *N,N*-diisopropylethylamine (Hünig's base).

Suitable aqueous buffers such as phosphate-buffered saline in pH range of 6 to 9.

Suitable solvents are polar aprotic solvents such as N,N-dimethylformamide or dimethylsulfoxide.

The reaction mixture containing the resulting radiolabeled oligonucleotide can be freed from the solvent and the crude can be dissolved in a suitable aqueous buffer solution for further purification.

The purification essentially comprises the steps chromatography, concentration and isolation applying techniques well known to the skilled in then art.

The chromatography is a preparatory HPLC typically with a C-18 reversed-phase column using aqueous and organic solvents as mobile phases.

The concentration of the fractions obtained from the chromatography can take place *via* a tangential flow filtration, particularly a diafiltration over a suitable membrane.

Finally, the isolation of the radiolabeled oligonucleotide from the eluent can typically take place by lyophilization.

For those radiolabeled oligonucleotides of the formula I wherein Q stands for the residue of the formula 2b the process comprises conjugating a thiol of formula V 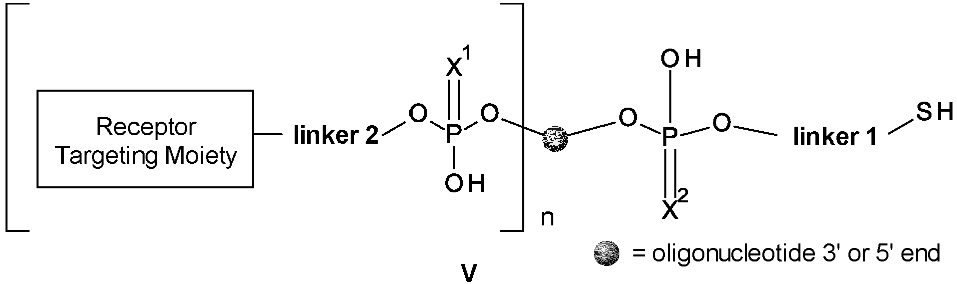 wherein,
X¹ and X² independently of each other are S or O;
linker 1 is a C₂₋₁₂- alkylene bridge, an ethylene glycol bridge containing 1 to 10 ethylene glycol units or a glycerol based bridge of the formula 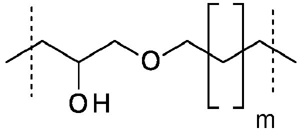
wherein m is an integer of 1 to 6;
linker 2 is an optionally amino group protected amino C₂₋₁₂-alkylene bridge, an amino ethylene glycol bridge containing 1 to 10 ethylene glycol units;
and the receptor targeting moiety is a moiety which adds additional functionality to the oligonucleotide;
with a radiolabeled maleimide compound of formula VI 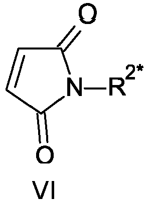
wherein R^{2*} is as above.

Radiolabeled maleimide derivatives are commercially available. The ³H labeled maleimide with R^{2*} methyl (Supplier 1) or ethyl (Supplier 2) can for instance be obtained from RC Tritec, Teufen, CH (Supplier 1), Pharmaron, Cardiff, UK (Supplier 2)
The conjugation reaction can be performed in the presence of an organic solvent at a reaction temperature of 0 °C to 50°C.

Suitable solvents are polar aprotic solvents such as *N*,*N*-dimethylformamide, dimethylsulfoxide or aqueous buffered systems.

The reaction mixture containing the resulting radiolabeled oligonucleotide can be freed form the solvent and the crude can be dissolved in a suitable aqueous buffer solution for further purification.

The purification essentially comprises the steps concentration and isolation applying techniques well known to the skilled in the art.

The concentration can take place *via* a tangential flow filtration, particularly a diafiltration of the aqueous solution over a suitable membrane.

The invention further comprises the use of the radiolabeled oligonucleotide for the determination of the biodistribution and pharmacokinetics of the oligonucleotide in the tissue or body fluid. In addition, tritium labeled oligonucleotides can be applied in bioscience, including quantitative whole body autoradiography (QWBA), target binding, and transporter efflux and uptake studies.

The invention also comprises a method for the determination of the biodistribution and pharmacokinetics of an oligonucleotide in the tissue or body fluid comprising
a) administering an effective amount of radiolabeled oligonucleotide of anyone of claims 1 to 11 to the tissue or the body fluid to be examined and
b) measuring the biodistribution and the pharmacokinetics of the radiolabeled oligonucleotide of anyone of claims 1 to 11 in the tissue or body fluid and optionally
c) imaging the radiolabeled oligonucleotide of anyone of claim 1 to 11 in the tissue or the body fluid to be examined by autoradiography.

The invention further comprises the oligonucleotide of the formula X 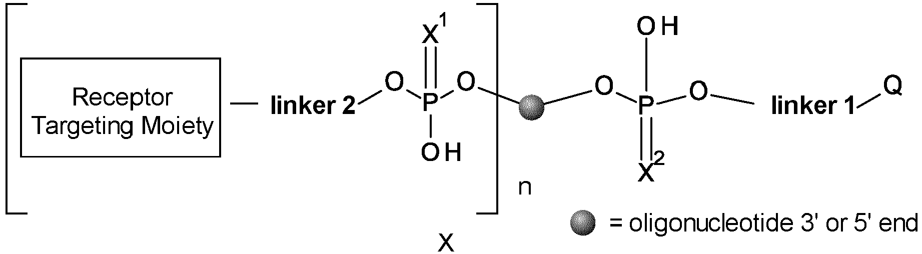 wherein,
n is 0 or 1;
X¹ and X² independently of each other are S or O;
linker 1 is a C₂₋₁₂- alkylene bridge, an ethylene glycol bridge containing 1 to 10 ethylene glycol units or a glycerol based bridge of the formula 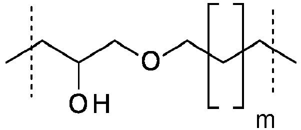
wherein m is an integer of 1 to 6;
linker 2 is an optionally amino group protected amino C₂₋₁₂-alkylene bridge, an amino ethylene glycol bridge containing 1 to 10 ethylene glycol units;
Q stands for a residue of the formula 2b' 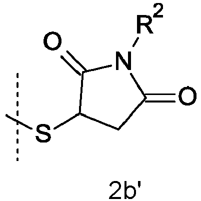
wherein R² is a C₁₋₆-alkyl group; and
the receptor targeting moiety is a moiety which adds additional functionality to the oligonucleotide.

The preferred embodiments described for the radiolabeled oligonucleotides of formula I likewise applies for the oligonucleotides of formula X.

Accordingly, R² stands for a C₁₋₄-alkyl group, preferably for a methyl or ethyl group more preferably for an ethyl group.

The preferred embodiments described for the radiolabeled oligonucleotides of formula Ib, Ic and Id likewise apply for the oligonucleotides of formula Xb 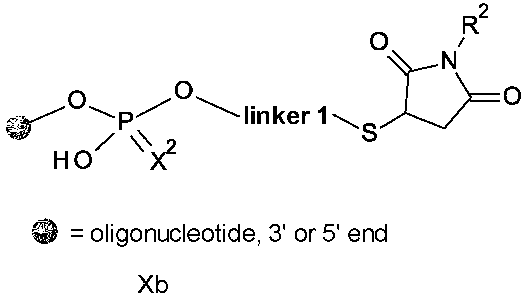
wherein R², X² and linker 1 are as above;
for the oligonucleotide of the formula Xc 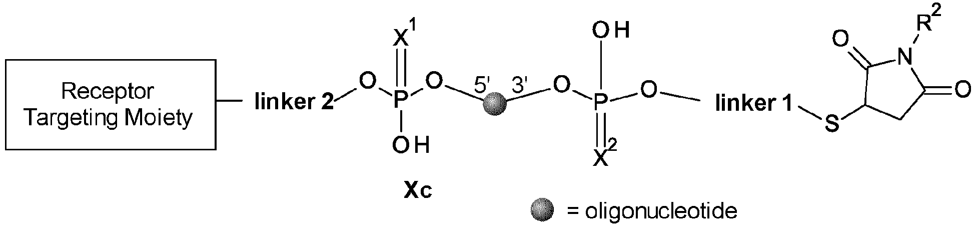
wherein R², X¹ and X², linker 1 and linker 2 are as above;
and
the receptor targeting moiety which is a non-nucleotide moiety, preferably a asialglycoprotein receptor targeting moiety, more preferably a GalNAc moiety of formula VII 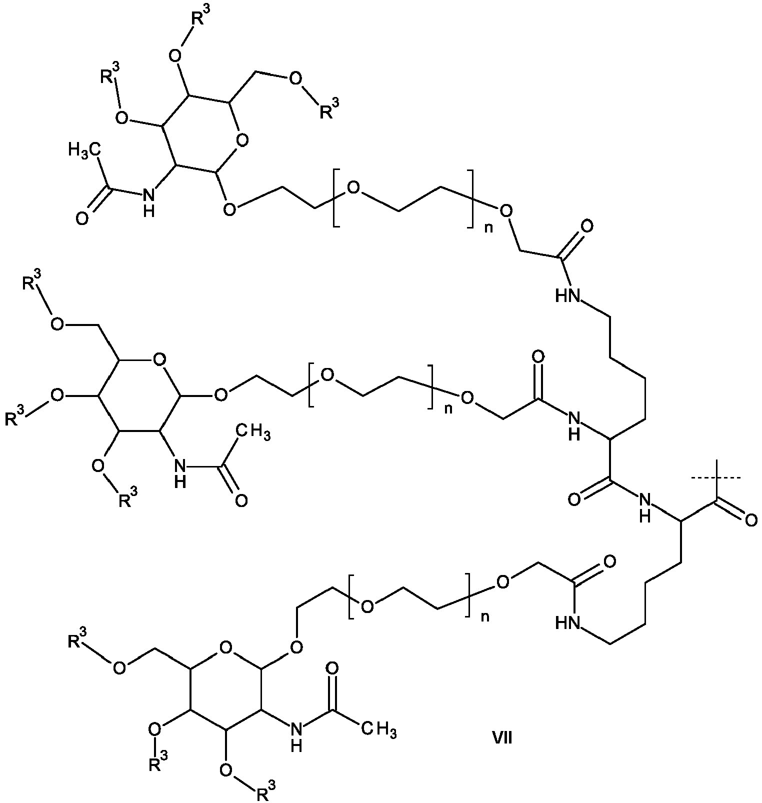 wherein R³ is hydrogen or a hydroxy protecting group and n is an integer from 0 to 10, preferably from 0 to 5, more preferably from 1 to 3, but most preferred is 2, corresponding salts, enantiomers and/ or a stereoisomer thereof.

The compounds disclosed herein have the following nucleobase sequence.
SEQ ID NO 1 : gcattggtattca (Oligo 1,3,5)
SEQ ID NO 2 : gagttacttgccaact (Oligo 2,6)
SEQ ID NO 3 : cagagttacttgccaact (Oligo 4)
SEQ ID NO 4 : ttacacttaattatacttcc (Oligo 7)

### Examples:

### Abbreviations:

- AcOH: acetic acid
- Bq: Becquerel
- Ci: curie
- Da: Dalton
- DI: deionized
- DIPEA: *N*,*N*-diisopropylethylamine (Hünig's base)
- DMAP: 4-(dimethylamino)-pyridine
- DMF: *N, N*-dimethylformamide
- DMSO: dimethylsulfoxide
- EtOH: ethanol
- GBB: glycerol based bridge
- h: hours
- HPLC: High-performance liquid chromatography
- *i*: iso
- LC-MS/MS: Liquid chromatography coupled to tandem mass spectrometry
- LNA: Locked nucleic acid
- LSC: Liquid scintillation counting
- MeOH: methanol
- min: minutes
- mM, nM: Millimolar, Nanomolar
- mL: Mililitre
- µL: Microlitre
- MS: mass spectrometry
- MW: molecular weight
- MWCO: molecular weight cut off
- *n*: normal
- NEM: *N*-ethyl maleimide
- ng: Nanogram
- nm: Nanometer
- NMM: *N*-methyl maleimide
- NSP: *N*-succinimidyl propionate
- p: para
- PBS: phosphate-buffered saline
- PCR: Polymerase chain reaction
- PD: Pharmacodynamic
- Prop: propionate
- QC: Quality Control (sample)
- QWBA: quantitative whole body autoradiography
- rpm: round per minutes
- rt: room temperature
- SRM: Selected reaction monitoring
- *t*: tertiary
- TEA: triethylamine
- UPLC: Ultra-performance liquid chromatography
- v: Volume

### General Methods:

All oligonucleotides, which were used as starting materials, were synthesized from Roche Pharma research and early development. Tritium labeled *N-*[³H]ethyl maleimide (specific activity: 2 TBq/mmol = 55 Ci/mmol) was obtained from Pharmaron (Cardiff, Wales, UK) as solution in pentane. Tritium labeled *N*-[³H]succinimidyl propionate (specific activity: 3.8 TBq/mmol = 103 Ci/mmol) was obtained from RC Tritec (Teufen, CH) as solution in toluene. Liquid scintillation counting for tritium compounds was accomplished using a HIDEX 300 SL and ULTIMATE GOLD cocktail (PerkinElmer Inc., Waltham, MA, USA). Reaction monitoring and purity for Oligos 1-3 were determined by HPLC Agilent 1210 at 260 nM wavelength, Waters XBridge RP18, 4.6 x 150 mm, 3.5 µm column at 60 °C ([A] = water/methanol/hexafluoro i-propanol/TEA : 950/25/21/2.3 mL; [B] = water/methanol/hexafluoro i-propanol/TEA : 175/800/21/2.3 mL) at flow 1.0 mL/min with the following gradient: 10% [B] to 60% [B] in 12 min. Oligos 4-6 were determined by UPLC Agilent 1290 at 260 nm wavelength, ACQUITY UPLC Oligonucleotide BEH C18, 2.1 x 50 mm, 1.7 µm column at 80 °C with same eluents and the following gradient: 10% [B] to 40% in 6 min. Oligo 7 was analyzed with same condition like Oligos 4-6 accept the following gradient: 10% [B] to 30% in 6 min. Mass spectrometry was perfomed by Waters Acquity UPLC H-class System equiped with Single Quadruple (SQ) and ESI Mass Detector Radiochemical purity was measured using the *β*-radioactivity HPLC detector RAMONA Quattro with internal solid scintillator (Raytest, Straubenhardt, Germany). Preparative HPLC for Oligos 1-3 were performed by Gilson PLC 2050 with XBridge C18 column, 5 µm, 10 mm × 250 mm and using water (950 mL)/methanol (25 mL)/TEA (2.3 mL)/hexafluoro i-propanol (21 mL) as mobile phase [A] and water (175 ml)/methanol (800 mL)/TEA (2.3 mL)/hexafluoro i-propanol (21 mL) as mobile phase [B] as gradient with 10% [B] to 60% [B] in 15 minutes. Concentration was determined by Eppendorf BioSprectrometer^{®} basic at 260 nm wavelength and the corresponding calculated molar extinction coefficient.

### Example 1 (non-radioactive conjugation on amine linker)

### a) Oligonucleotides used in the examples

5'-Am-C6*G*C*a*t*t*g*g*t*a*t*T*C*A; MW:4520.7 g/mol; (Oligo 1)
G*A*G*t*t*a*c*t*t*g*c*c*a*A*C*T*-Am-GBB; MW: 5506.5 g/mol; (Oligo 2)
G*C*a*t*t*g*g*t*a*t*T*C*A*-Am-GBB; MW: 4553.6 g/mol; (Oligo 3)

### b) General mode of reaction:

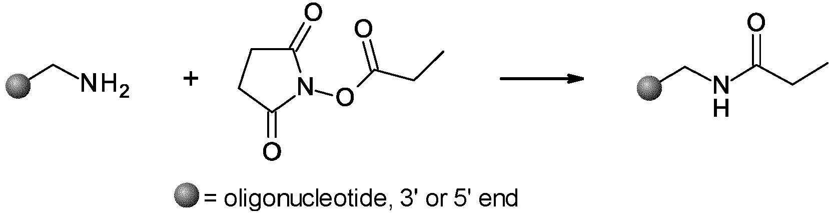

### c) General procedure

To 1 equivalent of oligo nucleotide, containing an amine linker on 5' or 3' end in DMF (volume factor: 125 mL/g) and 40 equivalent Hünig's base was added 1.2 equivalent N-succinimidyl propionate (NSP) to give a colorless suspension. The mixture stirred over night at room temperature to become a clear and colorless solution. The solvent was removed under high vacuum and the residue dissolved in PBS. Crude mixture was purified by preparative HPLC. The desired fractions were transferred into an Amicon^{®} Pro purification system (MWCO: 3.000 Da) and centrifuged at 4000 rpm. DI water was added and the process was repeated 4 times more to complete an exchange from HPLC eluent to water. The resulting aqueous solution was lyophilized to isolate the oligonucleotide as a colorless powder with a yield in range of 47% - 74% and 96% - 99% purity.

In accordance with the general procedure (1.c.) the oligonucleotides Oligo 1-3 have been conjugated.

### Example 1.d. (Conjugate 1)

Prop-5'-Am-C6*G*C*a*t*t*g*g*t*a*t*T*C*A; Yield: 47%, purity: 99%, MS (m/z): 4577.0 [M-(H)]⁻

### Example 1.e. (Conjugate 2)

G*A*G*t*t*a*c*t*t*g*c*c*a*A*C*T*-Am-GBB-Prop; Yield: 74%, purity: 95%, MS (m/z): 5561.6 [M-(H)]⁻

### Example 1.f. (Conjugate 3)

G*C*a*t*t*g*g*t*a*t*T*C*A*-Am-GBB-Prop; Yield: 60%, purity: 96%, MS (m/z): 4662.3 [M-(H)]⁻

### Example 2 (non-radioactive conjugation on thiol linker)

### a) Oligonucleotides used in the examples

5'-GN2-C6-caG*A*G*t*t*a*c*t*t*g*c*c*a*A*C*T*-C6SH; MW: 7709.5 g/mol; (Oligo 4)
G*C*a*t*t*g*g*t*a*t*T*C*A*-C6SH; MW: 4537.6 g/mol; (Oligo 5)
G*A*G*t*t*a*c*t*t*g*c*c*a*A*C*T*-C6SH; MW: 5491.5 g/mol; (Oligo 6)
5' -SH-C6*T*T* A *c* A *c*t*t*a*a*t*t*a*t*a*c*t*T*C*C; MW: 6742.3 g/mol; (Oligo 7)

### b) General mode of reaction:

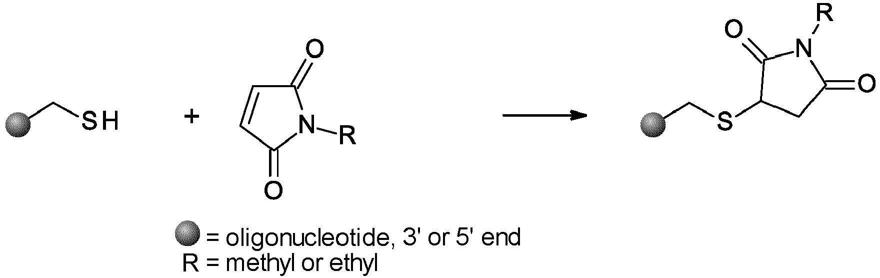

### c) General procedure

1 equivalent of oligonucleotide with 5' or 3' end sulfhydryl linker was dissolved in PBS (volume factor: 250 mL/g). 1.5 equivalent of *N*-alkylated maleimide (methyl or ethyl), dissolved in DMSO (volume factor: 1500 mL/g), was added to the aqueous solution and stirred at room temperature for 1 h. UPLC analysis showed a complete addition of maleimide to oligo nucleotide. To exchange the buffer to water, the reaction mixture was transferred into an Amicon^{®} Pro purification system (MWCO: 3.000 Da) and centrifuged at 4000 rpm. DI water was added and the process was repeated 4 times more to complete the exchange. The resulting aqueous solution was lyophilized to isolate the oligonucleotide as a colorless powder with a yield in range of 69% - 81% and 96% - 99% purity.

In accordance with the general procedure (2.c.) the oligonucleotides Oligo 4-7) have been conjugated.

### Example 2.d. (Conjugate 4)

5'-GN2-C6-caG*A*G*t*t*a*c*t*t*g*c*c*a*A*C*T*-C6SH-NEM; Yield: 73%, purity: 99%, MS (m/z): 7833.4 [M-(H)]⁻

### Example 2.e. (Conjugate 5)

G*C*a*t*t*g*g*t*a*t*T*C*A*-C6SH-NEM; Yield: 73%, purity: 99%, MS (m/z): 4662.3 [M-(H)]⁻

### Example 2.f. (Conjugate 6)

G*C*a*t*t*g*g*t*a*t*T*C*A*-C6SH-NMM; Yield: 81%, purity: 99%, MS (m/z): 4648.2 [M-(H)]⁻

### Example 2.g. (Conjugate 7)

G*A*G*t*t*a*c*t*t*g*c*c*a*A*C*T*-C6SH-NEM; Yield: 73%, purity: 96%, MS (m/z): 5616.2 [M-(H)]⁻. ¹H NMR (600 MHz, D₂O) δ ppm 4.11 (br s, 2 H), 4.05 - 4.17 (m, 1 H), 3.75 (br s, 2 H), 3.43 - 3.52 (m, 1 H), 2.80 - 2.91 (m, 1 H), 2.74 - 2.91 (m, 2 H), 1.71 - 1.83 (m, 2 H), 1.61 - 1.77 (m, 2 H), 1.44 - 1.59 (m, 2 H), 1.43 - 1.56 (m, 2 H), 1.34 (br s, 3 H)
NMR data limited to linker and NEM conjugated label.

### Example 2.h. (Conjugate 8)

G*A *G*t*t*a*c*t*t*g*c*c*a*A*C*T*-C6SH-NMM; Yield: 69%, purity: 97%, MS (m/z): 5602.2 [M-(H)]⁻

### Example 2.i. (Conjugate 9)

5'-NEM-SH- C6*T*T*A*c*A*c*t*t*a*a*t*t*a*t*a*c*t*T*C*C; Yield: 97%, purity: 99%, MS (m/z): 6868.7 [M-(H)]⁻

### Example 3 (Radioactive conjugation oligonucleotides)

### Example 3.a. ([³H]-Compound 1 based on conjugate 2)

**G*A*G*t*t*a*c*t*t*g*c*c*a*A*C*T*-Am-GBB-[³H]-Prop**

370 MBq (10 mCi) of *N*-[³H]succinimidyl propionate (17.3 µg, 0.079 µmol) with a specific activity of 3.811 GBq/mmol (103 Ci/mmol) and dissolved in 2 mL toluene was diluted with 22.8 µg of the corresponding non-radioactive N succinimidyl propionate to achieve a total amount of 40.1 µg (0.234 µmol) with a specific activity of 1.554 TBq/mmol (42 Ci/mmol). The solvent was removed by evaporation and the solid residue was dissolved in 100 µl DMF. 0.98 mg (0.167 µmol) of Olio 2, dissolved in 250 µL DMF and 1.3 µL (0.97 µmol) DIPEA, was dropped to the [³H]NSP solution and stirred over night at room temperature. UPLC showed a conversion of 40% to the desired product. The reaction solution was filled into an Amicon^{®} Pro purification system (MWCO: 3.000 Da) and centrifuged at 4000 rpm to change the solvent to water/methanol/hexafluoro i-propanol/TEA : 950/25/21/2.3 for preparative HPLC sample preparation. After prep-HPLC, the corresponding fraction was deluted with PBS and transferred into an Amicon^{®} Pro purification system (MWCO: 3.000 Da) and centrifuged at 4000 rpm. PBS was added and the process was repeated 4 times more to achieve a chemical purity of 99%. Volume: 0.55 mL, concentration: 0.32 mg/mL, amount: 0.19 mg (yield: 19.5%), activity: 51.8 MBq (1.4 mCi), specific activity: 262.7 MBq/mg (7.1 mCi/mg) which is equal to 1.554 TBq/mmol (42 Ci/mmol).

### Example 3.b. ([³H]-Compound 2 based on conjugate 4)

**5'-GN2-C6-caG*A*G*t*t*a*c*t*t*g*c*c*a*A*C*T*-C6SH-[³H]-NEM**

370 MBq (10 mCi) of *N-*[³H]ethyl maleimide (20.5 µg, 0.159 µmol) in 4 mL pentane was concentrated on a silica gel pre-packed column and eluted with 2x 0.5 mL DMSO. A solution of Oligo 4 (1.02 mg, 0.132 µmol) in 1 mL PBS was added and stirred 1h at room temperature. UPLC analysis showed 20% of the desired product. Non-radioactive NEM (166 µg, 1.32 µmol) was added and stirred at room temperature for 1 h. HPLC showed a complete addition to the desired product. The reaction solution was transferred into a 5 mL Float-A-Lyzer^{®} tube (MWCO: 500 - 1000 Da) and dialyzed against PBS pH 7.1 at room temperature. Buffer was changed 4 times after 45 minutes and stored overnight in the fridge. UPLC showed a radio chemical purity of 93%. Volume: 2.9 mL, concentration: 0.33 mg/mL, amount: 0.95 mg (yield: 92%), activity: 33.7 MBq (0.91 mCi), specific activity: 35.5 MBq/mg (953 µCi/mg) which is equal to 0.3 TBq/mmol (7.9 Ci/mmol).

### Example 3.c. ([³H]-Compound 3 based on conjugate 7)

G*A*G*t*t*a*c*t*t*g*c*c*a*A*C*T*-C6SH-[³H]-NEM

1.1 GBq (30 mCi) of *N*-[³H]ethyl maleimide (61.5 µg, 0.477 µmol) in 12 mL pentane was concentrated on a silica gel pre-packed column and eluted with 2x 0.5 mL DMSO. A solution of Oligo 6 (2.20 mg, 0.401µmol) in 1 mL PBS was added and stirred 1 h at room temperature. UPLC analysis showed 40% of the desired product. Non-radioactive NEM (502 µg, 4.01 µmol) was added and stirred at room temperature for 1 h. HPLC showed a complete addition to the desired product. The reaction solution was transferred into a 5 mL Float-A-Lyzer^{®} tube (MWCO: 500 - 1000 Da) and dialyzed against PBS pH 7.1 at room temperature. Buffer was changed 4 times after 45 minutes and stored overnight in the fridge. UPLC showed a high polar radio impurity. The solution was filled into an Amicon^{®} Pro purification system (MWCO: 3.000 Da) and centrifuged at 4000 rpm. PBS was added and the process was repeated 4 times more to achieve a chemical purity of 99%. Volume: 1.0 mL, concentration: 1.58 mg/mL, amount: 1.58 mg (yield: 70%), activity: 163 MBq (4.4 mCi), specific activity: 104 MBq/mg (2.8 mCi/mg) which is equal to 614 MBq/mmol (16.6 Ci/mmol).

### Example 3.d. ([³H]-Compound 4 based on conjugate 9)

**5'-[³H]-NEM-SH-C6***T*T*A*c*A*c*t*t*a*a*t*t*a*t*a*c*t*T*C*C

370 MBq (10 mCi) of *N*-[³H]ethyl maleimide (20.5 µg, 0.159 µmol) in 4 mL pentane was concentrated on a silica gel pre-packed column and eluted with 2x 0.5 mL DMSO, dropped into a solution of Oligo 7 (1.13 mg, 0.168 µmol) in 0.5 mL PBS and let it stir for 1.5 h at rt. UPLC showed 45% desired product and 55% starting material. Non-radioactive NEM (210 µg, 1.68 µmol) was added and stirred at room temperature for 1 h. HPLC showed a complete addition to the desired product. The reaction solution was transferred into an Amicon^{®} Pro purification system (MWCO: 3.000 Da) and centrifuged at 4000 rpm. PBS was added and the process was repeated 4 times more to achieve a chemical purity of 99%. Volume: 1.0 mL, concentration: 1.80 mg/mL, amount: 1.07 mg (yield: 93%), activity: 71 MBq (1.91 mCi), specific activity: 67 MBq/mg (1.8 mCi/mg) which is equal to 481 MBq/mmol (13.0 Ci/mmol).

### Tissue Exposure Study of unlabeled and tritium labeled LNA - A feasibility Study

The studies have been performed with the following compounds:
5'-GN2-C6-ca G*A*G*t*t*a*c*t*t*g*c*c*a*A*C*T*-3' (GalNAc LNA study compound A)
5'- G*A*G*t*t*a*c*t*t*g*c*c*a*A*C*T*-3' (LNA study compound A)
5'-GN2-C6-caG*A*G*t*t*a*c*t*t*g*c*c*a*A*C*T*-C6SH-[³H]-NEM (=Example 3.b)
5'-G*A*G*t*t*a*c*t*t*g*c*c*a*A*C*T*-C6SH-[³H]-NEM (=Example 3.c)

A single dose PK experiment with Example 3.b ([³H]-compound 2 based on conjugate 4) and Example 3.c ([³H]-compound 3 based on conjugate 7) at 1 mg/kg was done. LNAs were analyzed in liver 24, 72 and 336 h after dosing. The study will confirm the feasibility of oligonucleotides with radioactive conjugation.

### Experimental part for in vivo study:

### Preparation of stock solution, calibration standards and quality checks

Serial dilutions were made from stock solution (approx. 1 mg/mL in PCR grade water, the exact concentration of the stock solution will be quantified with the spectro-photometric device Nanodrop (Thermo Scientific) based on the extinction coefficient at 260 nm) to generate working solutions in water from approx. 100 ng/mL up to approx. 250000 ng/mL.

These working solutions were used to spike plasma following this procedure: 1 µL working solution was added to 49 µL plasma in order to create calibration samples, and quality control samples at 4 concentration levels in plasma.

### Extraction method

Calibration standards and quality control samples (freshly prepared in plasma, 50 µL) were treated for protein denaturation with 150 µL of 4 M guanidine thiocyanate after addition of the internal standard. After vigorously mixing (20 min at 1600 rpm), 200 µL of a water/hexafluoroisopropanol/diisopropylethylamine solution (100:4:0.2, v/v/v) were added, followed by mixing (15 min at 1500 rpm). Then a clean-up step was performed by means of solid-phase-extraction cartridges (Waters, OASIS HLB 5 mg, 30 µm) after elution and evaporation to dryness (30 - 45 min at +40 °C) the samples were reconstituted in 200 µL of mobile phase (water/methanol/hexafluoroisopropanol/diisopropylethylamine (95/5/1/0.2, v/v/v/v)). After vortex mixing (10 min at 1500 rpm), an aliquot (20 µL) was injected into a LC-MS/MS system (50 µL loop).

### Description of LC-MS/MS method

A Shimadzu 30ADXR pump was used, equipped with a Waters Acquity C18 column (50 x 2.1 mm) at 60 °C. The analytes and internal standard were separated from matrix interferences using gradient elution from water/methanol/hexafluoroisopropanol/diisopropyletylamine (95/5/1/0.2, v/v/v/v) to water/methanol/hexafluoroisopropanol/diisopropyletylamine (10/90/1/0.2, v/v/v/v) within 4.0 min at a flow rate of 0.4 mL/min.

Mass spectrometric detection was carried out on an AB-Sciex Triple Quad 6500⁺ mass spectrometer using SRM in the negative ion mode.

### Liquid scintillation counting

A Packard Tri-carb 3100TR was used for LSC analysis.

### Detailed description of the figures:

In Fig. 1 the liver concentration of a GalNAc LNA study compound A (dotted line) and the LNA study compound A without GalNAc (continuous line) have been analyzed by LC-MS/MS. The GalNAc labeled LNA shows as expected a high initial uptake in the liver plasma and a normal decrease over the time. Likewise shows the naked, i.e. not GalNAc containing LNA, a lower level of uptake.

In Fig. 2 the liver concentration of the tritium labeled compounds of Example 3.b (dotted line) and Example 3.c (continuous line) have been analyzed by LSC. This figure shows, that the radiolabeled GalNAc compound, despite of the maleimide conjugation, has an equivalent liver uptake as a therapeutic GalNAc LNA (Fig.1).

PD effects are comparable for the unlabeled and radio labeled oligonucleotide. LNA concentration measurements in the liver of the radioactivity by LSC is similar to the therapeutic LNAs, determined by LC-MS/MS.

Figure 2 impressively illustrates the high specificity of the radiolabeled oligonucleotide compounds of the present invention.

## Claims

1. Radiolabeled oligonucleotide of the formula I 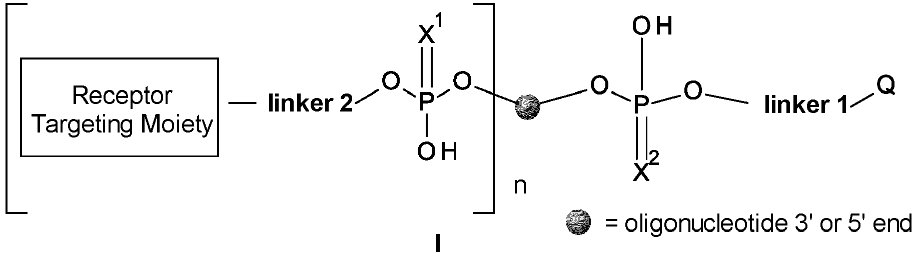 wherein,
n is 0 or 1;
X¹ and X² independently of each other are S or O;
linker 1 is a C₂₋₁₂- alkylene bridge, an ethylene glycol bridge containing 1 to 10 ethylene glycol units or a glycerol based bridge of the formula 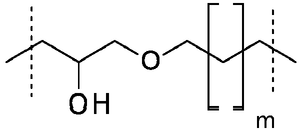
wherein m is an integer of 1 to 6;
linker 2 is an optionally amino group protected amino C₂₋₁₂-alkylene bridge, an amino ethylene glycol bridge containing 1 to 10 ethylene glycol units;
Q stands for a residue of the formula 2a or 2b 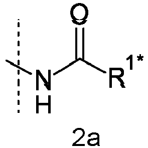 or 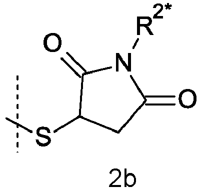
wherein R^{1*} and R^{2*} are C₁₋₆-alkyl labelled with ³H- or ¹⁴C-; and
the receptor targeting moiety is a moiety which adds additional functionality to the oligonucleotide.

2. Radiolabeled oligonucleotide of claim 1, wherein Q has the formula 2b and the conjugation is at the 3' or 5' end of the oligonucleotide.

3. Radiolabeled oligonucleotide of claim 1or 2, wherein Q has the formula 2a and the conjugation is at the 3' or 5' end of the oligonucleotide.

4. Radiolabeled oligonucleotide of anyone of claims 1 to 3, wherein R^{1*} and R^{2*} is C₁₋₄-alkyl labelled with ³H- or ¹⁴C, preferably methyl or ethyl labelled with ³H- or ¹⁴C.

5. Radiolabeled oligonucleotide of anyone of claims 1 to 4, wherein the labeling is with ³H.

6. Radiolabeled oligonucleotide of anyone of claims 1 to 5, wherein the oligonucleotide comprises a contiguous nucleotide sequence of 7 to 30 nucleotides consisting of optionally modified DNA, RNA or LNA nucleoside monomers or combinations thereof.

7. Radiolabeled oligonucleotide of claim 1 of the formula Ib 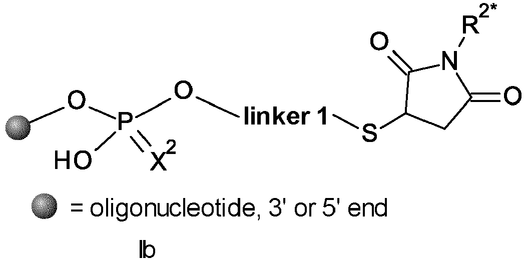 wherein R^{2*}, X² and linker 1 are as defined in claim 1

8. Radiolabeled oligonucleotide of claim 1 of the formula Ic 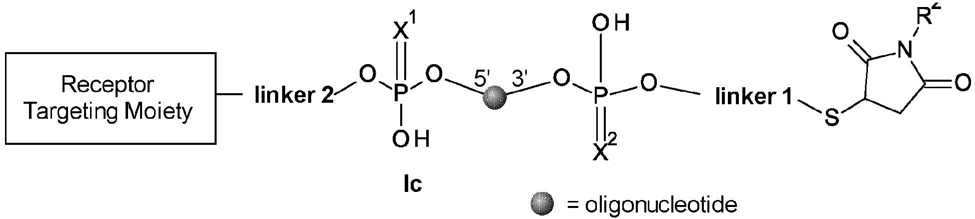 wherein R^{2*}, X¹ and X², linker 1 and linker 2 are as defined in claim 1.

9. Radiolabeled oligonucleotide of claim 1, wherein the receptor targeting moiety is a non-nucleotide moiety, preferably a asialglycoprotein receptor targeting moiety, more preferably a GalNAc moiety of formula VII 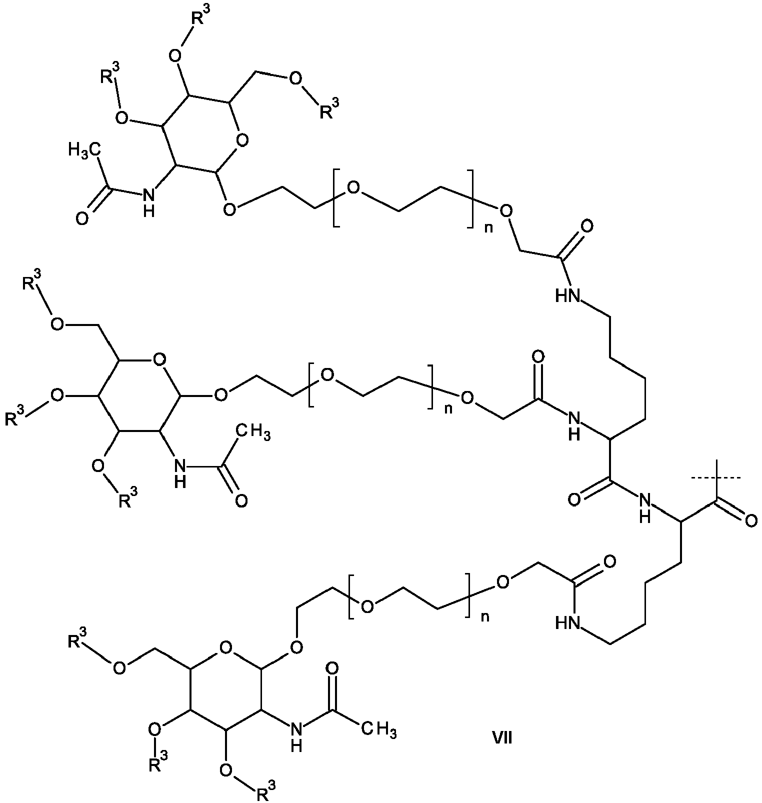 wherein R³ is hydrogen or a hydroxy protecting group and n is an integer from 0 to 10, preferably from 0 to 5, more preferably from 1 to 3, but most preferred is 2, corresponding salts, enantiomers and/ or a stereoisomer thereof.

10. Radiolabeled oligonucleotide of claim 1 of the formulae Id 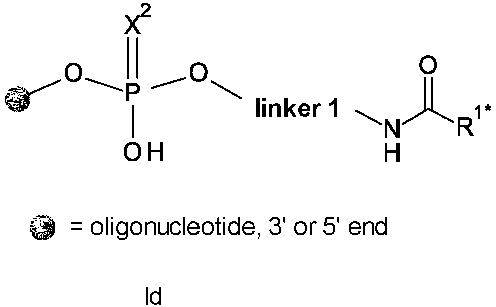 wherein R^{1*}, X² and linker 1 are as defined in claim 1.

11. Radiolabeled oligonucleotide of anyone of claims 1 to 10 having a specific activity of 37 GBq/mmol (1 Ci/mmol) to 3.7 TBq/mmol (100 Ci/mmol), preferably of 111 GBq/mmol (3 Ci/mmol) to 1.85 TBq/mmol (50 Ci/mmol), more preferably of 185 GBq/mmol (5 Ci/mmol) to 740 GBq/mmol (20 Ci/mmol).

12. Process for the preparation of a radiolabeled oligonucleotide of the formula I according to claim 1, wherein Q stands for the residue of the formula 2a comprising conjugating an amine of formula III 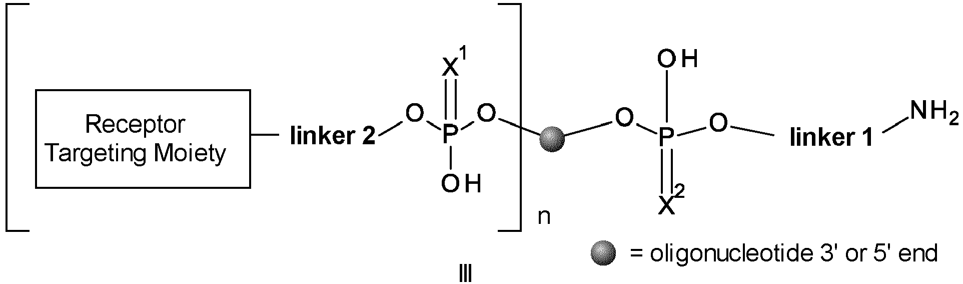 wherein,
n is 0 or 1;
X¹ and X² independently of each other are S or O;
linker 1 is a C₂₋₁₂- alkylene bridge, an ethylene glycol bridge containing 1 to 10 ethylene glycol units or a glycerol based bridge of the formula 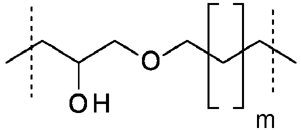
wherein m is an integer of 1 to 6;
linker 2 is an optionally amino group protected amino C₂₋₁₂- alkylene bridge, an amino ethylene glycol bridge containing 1 to 10 ethylene glycol units;
the receptor targeting moiety is a non-nucleotide moiety which adds additional functionality to the oligonucleotide, particularly an asialglycoprotein receptor targeting moiety, preferably a GalNAc moiety;
with a radiolabeled succinimide compound of formula IV 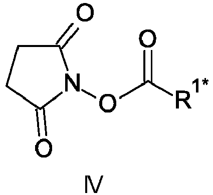
wherein R^{1*} is as defined in claim 1.

13. Process for the preparation of a radiolabeled oligonucleotide of the formula I according to claim 1, wherein Q stands for the residue of the formula 2b comprising conjugating a thiol of formula V 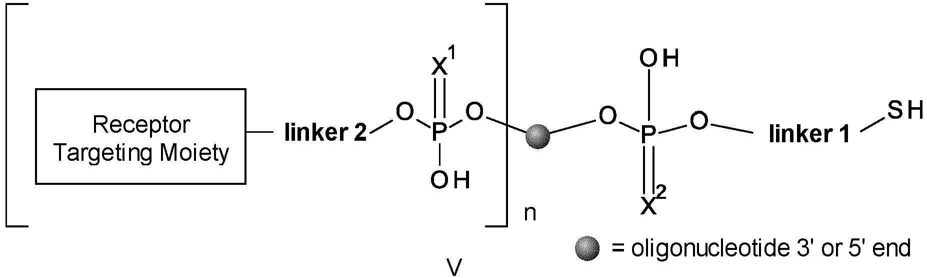 wherein,
n is 0 or 1;
X¹ and X² independently of each other are S or O;
linker 1 is a C₂₋₁₂- alkylene bridge, an ethylene glycol bridge containing 1 to 10 ethylene glycol units or a glycerol based bridge of the formula 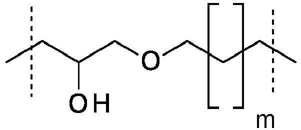
wherein m is an integer of 1 to 6;
linker 2 is an optionally amino group protected amino C₂₋₁₂- alkylene bridge, an amino ethylene glycol bridge containing 1 to 10 ethylene glycol units;
the receptor targeting moiety is a non-nucleotide moiety which adds additional functionality to the oligonucleotide, particularly an asialglycoprotein receptor targeting moiety, preferably a GalNAc moiety;
with a radiolabeled maleinimide compound of formula VI 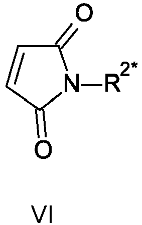
wherein R^{2*} is as defined in claim 1.

14. Use of the radiolabeled oligonucleotide of anyone of claims 1 to 11 for the determination of the biodistribution and pharmacokinetics of the oligonucleotide in a tissue or body fluid.

15. Method for the determination of the biodistribution and pharmacokinetics of an oligonucleotide in a tissue or body fluid comprising;
a) administering an effective amount of radiolabeled oligonucleotide of anyone of claims 1 to 11 to a tissue or the body fluid to be examined and
b) measuring the biodistribution and the pharmacokinetics of the radiolabeled oligonucleotide of anyone of claims 1 to 11 in a tissue or body fluid and optionally
c) imaging the radiolabeled oligonucleotide of anyone of claim 1 to 11 in a tissue or the body fluid to be examined by autoradiography.

16. Oligonucleotide of the formula X 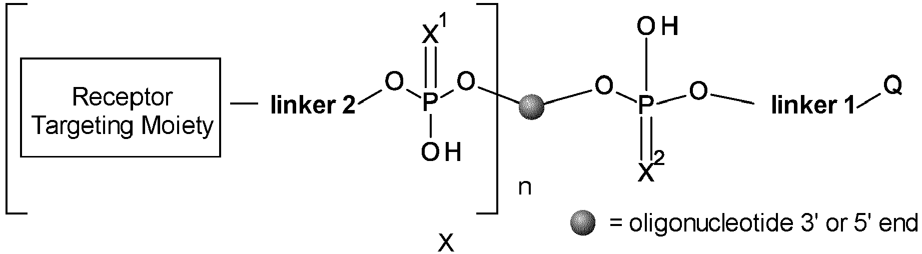 wherein,
n is 0 or 1;
X¹ and X² independently of each other are S or O;
linker 1 is a C₂₋₁₂- alkylene bridge, an ethylene glycol bridge containing 1 to 10 ethylene glycol units or a glycerol based bridge of the formula 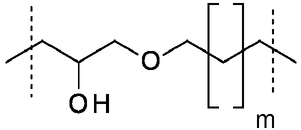
wherein m is an integer of 1 to 6;
linker 2 is an optionally amino group protected amino C₂₋₁₂-alkylene bridge, an amino ethylene glycol bridge containing 1 to 10 ethylene glycol units;
Q stands for a residue of the formula 2b' 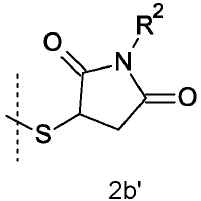
wherein R² is a C₁₋₆-alkyl group; and
the receptor targeting moiety is a moiety which adds additional functionality to the oligonucleotide.

17. Oligonucleotide of claim 16, wherein R² is a C₁₋₄-alkyl group, preferably a methyl or ethyl group.

18. Oligonucleotide of anyone of claim 16 or 17, wherein the oligonucleotide comprises a contiguous nucleotide sequence of 7 to 30 nucleotides consisting of optionally modified DNA, RNA or LNA nucleoside monomers or combinations thereof.

19. Oligonucleotide of claim 16 of the formula Xb 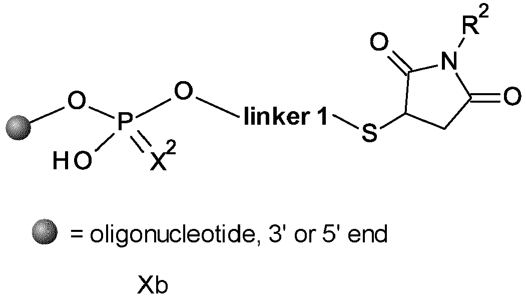 wherein R², X² and linker 1 are as defined in claim 16.

20. Oligonucleotide of claim 16 of the formula Xc 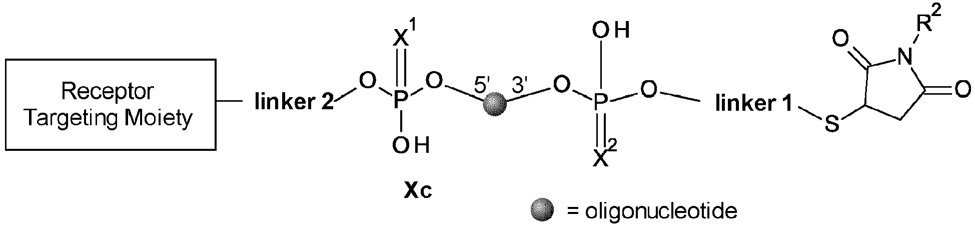 wherein R², X¹ and X², linker 1 and linker 2 are as defined in claim 16.

21. Oligonucleotide of claim 16, wherein the receptor targeting moiety is a non-nucleotide moiety, preferably a asialglycoprotein receptor targeting moiety, more preferably a GalNAc moiety of formula VII 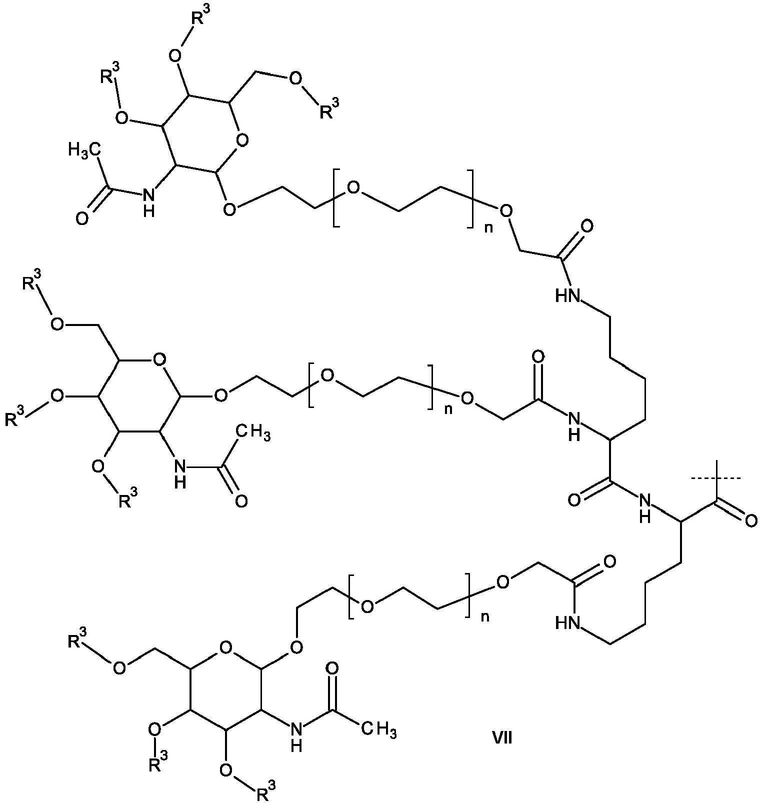 wherein R³ is hydrogen or a hydroxy protecting group and n is an integer from 0 to 10, preferably from 0 to 5, more preferably from 1 to 3, but most preferred is 2, corresponding salts, enantiomers and/ or a stereoisomer thereof.

## Patentansprüche

1. Radioaktiv markiertes Oligonukleotid der Formel I 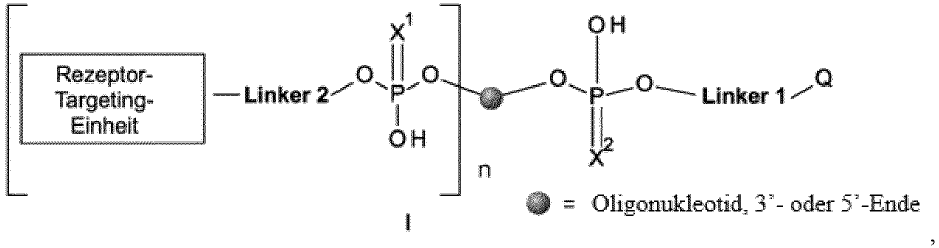 wobei
n 0 oder 1 ist;
X¹ und X² unabhängig voneinander S oder O sind;
Linker 1 eine C₂₋₁₂-Alkylenbrücke, eine Ethylenglykolbrücke, enthaltend 1 bis 10 Ethylenglykoleinheiten, oder eine Glycerol-basierte Brücke der Formel 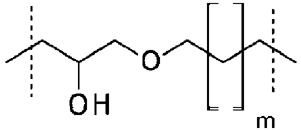
ist, wobei m eine ganze Zahl von 1 bis 6 ist;
Linker 2 eine gegebenenfalls aminogruppengeschützte Amino-C₂₋₁₂-alkylenbrücke, eine Aminoethylenglykolbrücke, enthaltend 1 bis 10 Ethylenglykoleinheiten, ist;
Q für einen Rest der Formel 2a oder 2b steht 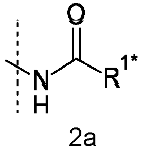 oder 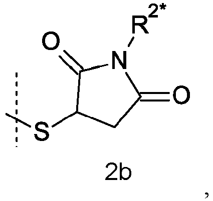
wobei R^{1*} und R^{2*} mit ³H- oder ¹⁴C- markiertes C₁₋₆-Alkyl sind; und
die Rezeptor-Targeting-Einheit eine Einheit ist, die dem Oligonukleotid zusätzliche Funktionalität verleiht.

2. Radioaktiv markiertes Oligonukleotid nach Anspruch 1, wobei Q die Formel 2b aufweist und die Konjugation am 3'- oder 5'-Ende des Oligonukleotids vorliegt.

3. Radioaktiv markiertes Oligonukleotid nach Anspruch 1 oder 2, wobei Q die Formel 2a aufweist und die Konjugation am 3'- oder 5'-Ende des Oligonukleotids vorliegt.

4. Radioaktiv markiertes Oligonukleotid nach einem der Ansprüche 1 bis 3, wobei R^{1*} und R^{2*} mit ³H- oder ¹⁴C markiertes C₁₋₄-Alkyl, vorzugsweise mit ³H- oder ¹⁴C markiertes Methyl oder Ethyl sind.

5. Radioaktiv markiertes Oligonukleotid nach einem der Ansprüche 1 bis 4, wobei die Markierung mit ³H erfolgt.

6. Radioaktiv markiertes Oligonukleotid nach einem der Ansprüche 1 bis 5, wobei das Oligonukleotid eine zusammenhängende Nukleotidsequenz aus 7 bis 30 Nukleotiden, bestehend aus gegebenenfalls modifizierten DNA-, RNA- oder LNA-Nukleosidmonomeren oder Kombinationen davon, umfasst.

7. Radioaktiv markiertes Oligonukleotid nach Anspruch 1 der Formel Ib 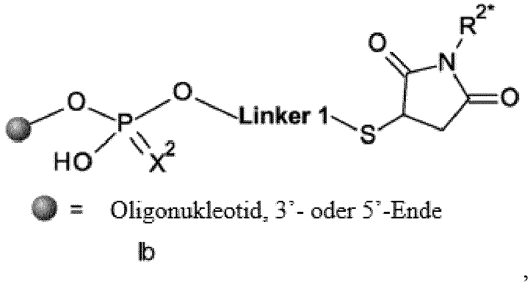 wobei R^{2*}, X² und Linker 1 wie in Anspruch 1 definiert sind.

8. Radioaktiv markiertes Oligonukleotid nach Anspruch 1 der Formel Ic 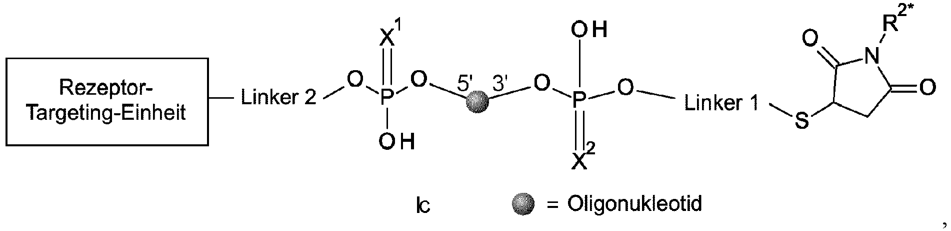 wobei R^{2*}, X¹ und X², Linker 1 und Linker 2 wie in Anspruch 1 definiert sind.

9. Radioaktiv markiertes Oligonukleotid nach Anspruch 1, wobei die Rezeptor-Targeting-Einheit eine Nicht-Nukleotideinheit, vorzugsweise eine Asialoglykoproteinrezeptor-Targeting-Einheit, stärker bevorzugt eine GalNAc-Einheit der Formel VII 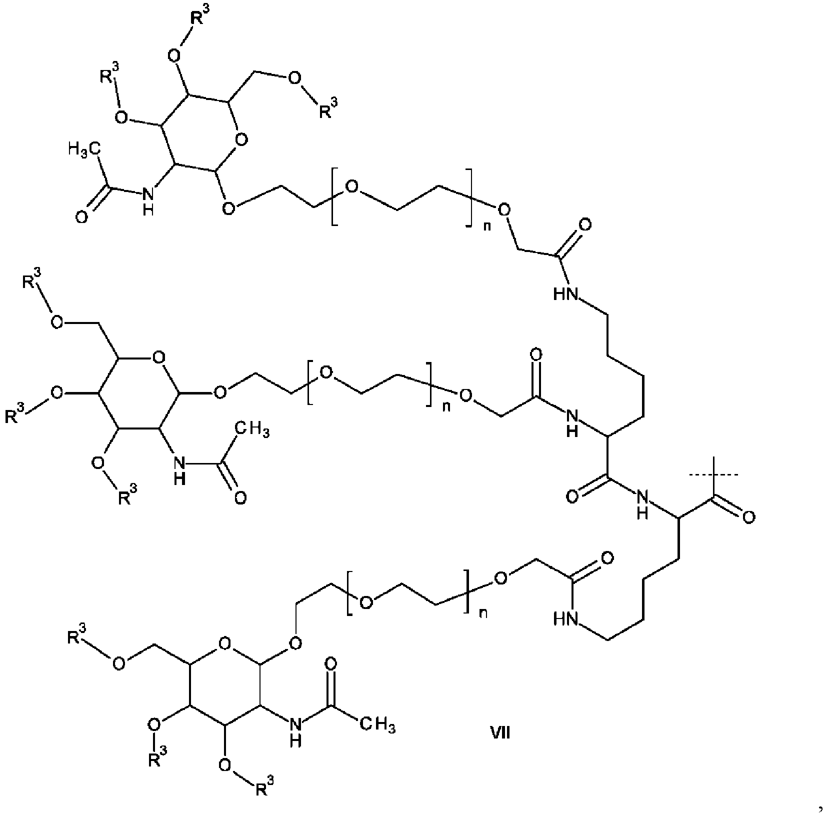 wobei R³ Wasserstoff oder eine Hydroxyschutzgruppe ist und n eine ganze Zahl von 0 bis 10, vorzugsweise von 0 bis 5, stärker bevorzugt von 1 bis 3, am stärksten bevorzugt aber 2 ist, entsprechende Salze, Enantiomere und/oder ein Stereoisomer davon ist.

10. Radioaktiv markiertes Oligonukleotid nach Anspruch 1 der Formel Id 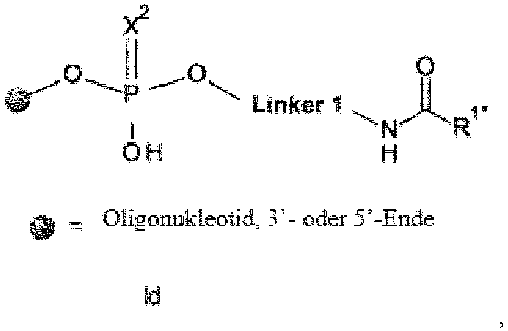 wobei R^{1*}, X² und Linker 1 wie in Anspruch 1 definiert sind.

11. Radioaktiv markiertes Oligonukleotid nach einem der Ansprüche 1 bis 10 mit einer spezifischen Aktivität von 37 GBq/mmol (1 Ci/mmol) bis 3,7 TBq/mmol (100 Ci/mmol), vorzugsweise von 111 GBq/mmol (3 Ci/mmol) bis 1,85 TBq/mmol (50 Ci/mmol), stärker bevorzugt von 185 GBq/mmol (5 Ci/mmol) bis 740 GBq/mmol (20 Ci/mmol).

12. Verfahren zur Herstellung eines radioaktiv markierten Oligonukleotids der Formel I nach Anspruch 1, wobei Q für den Rest der Formel 2a steht, umfassend das Konjugieren eines Amins der Formel III 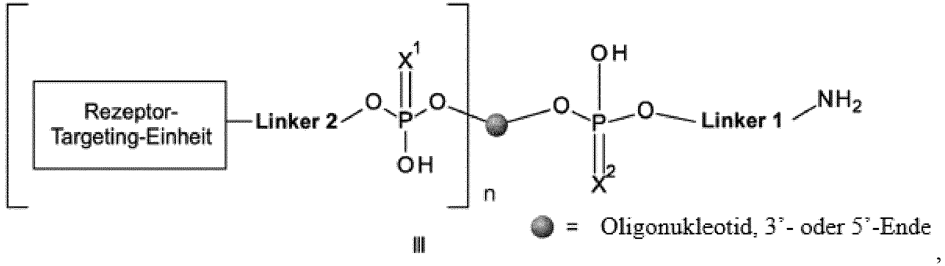 wobei
n 0 oder 1 ist;
X¹ und X² unabhängig voneinander S oder O sind;
Linker 1 eine C₂₋₁₂-Alkylenbrücke, eine Ethylenglykolbrücke, enthaltend 1 bis 10 Ethylenglykoleinheiten, oder eine Glycerol-basierte Brücke der Formel 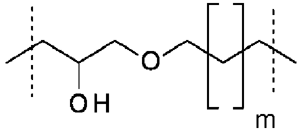
ist, wobei m eine ganze Zahl von 1 bis 6 ist;
Linker 2 eine gegebenenfalls aminogruppengeschützte Amino-C₂₋₁₂-alkylenbrücke, eine Aminoethylenglykolbrücke, enthaltend 1 bis 10 Ethylenglykoleinheiten, ist;
die Rezeptor-Targeting-Einheit eine Nicht-Nukleotideinheit, die dem Oligonukleotid zusätzliche Funktionalität verleiht, insbesondere eine Asialoglykoproteinrezeptor-Targeting-Einheit, vorzugsweise eine GalNAc-Einheit ist;
mit einer radioaktiv markierten Succinimidverbindung der Formel IV 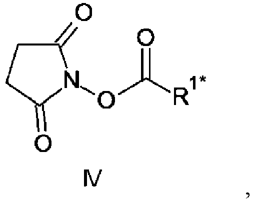
wobei R^{1*} wie in Anspruch 1 definiert ist.

13. Verfahren zur Herstellung eines radioaktiv markierten Oligonukleotids der Formel I nach Anspruch 1, wobei Q für den Rest der Formel 2b steht, umfassend das Konjugieren eines Thiols der Formel V 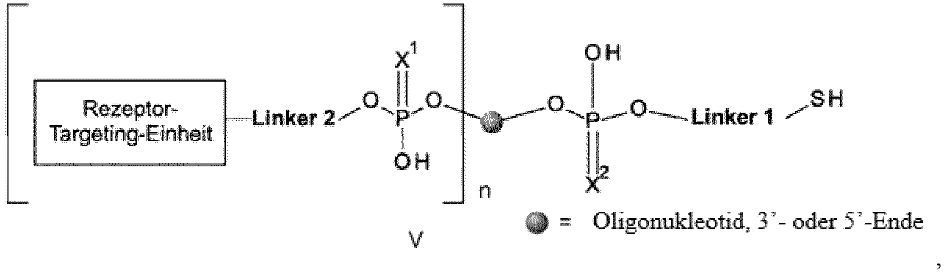 wobei
n 0 oder 1 ist;
X¹ und X² unabhängig voneinander S oder O sind;
Linker 1 eine C₂₋₁₂-Alkylenbrücke, eine Ethylenglykolbrücke, enthaltend 1 bis 10 Ethylenglykoleinheiten, oder eine Glycerol-basierte Brücke der Formel 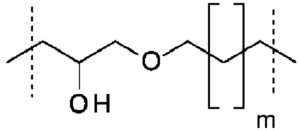
ist, wobei m eine ganze Zahl von 1 bis 6 ist;
Linker 2 eine gegebenenfalls aminogruppengeschützte Amino-C₂₋₁₂-alkylenbrücke, eine Aminoethylenglykolbrücke, enthaltend 1 bis 10 Ethylenglykoleinheiten, ist;
die Rezeptor-Targeting-Einheit eine Nicht-Nukleotideinheit, die dem Oligonukleotid zusätzliche Funktionalität verleiht, insbesondere eine Asialoglykoproteinrezeptor-Targeting-Einheit, vorzugsweise eine GalNAc-Einheit ist;
mit einer radioaktiv markierten Maleimidverbindung der Formel VI 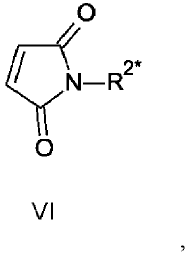
wobei R²* wie in Anspruch 1 definiert ist.

14. Verwendung des radioaktiv markierten Oligonukleotids nach einem der Ansprüche 1 bis 11 zur Bestimmung der Bioverteilung und Pharmakokinetik des Oligonukleotids in einem Gewebe oder einer Körperflüssigkeit.

15. Verfahren zur Bestimmung der Bioverteilung und Pharmakokinetik eines Oligonukleotids in einem Gewebe oder einer Körperflüssigkeit, umfassend:
a) Verabreichen einer wirksamen Menge eines radioaktiv markierten Oligonukleotids nach einem der Ansprüche 1 bis 11 an ein zu untersuchendes Gewebe oder die zu untersuchende Körperflüssigkeit; und
b) Messen der Bioverteilung und Pharmakokinetik des radioaktiv markierten Oligonukleotids nach einem der Ansprüche 1 bis 11 in einem Gewebe oder einer Körperflüssigkeit und gegebenenfalls
c) Bildgeben des radioaktiv markierten Oligonukleotids nach einem der Ansprüche 1 bis 11 in einem zu untersuchenden Gewebe oder der zu untersuchenden Körperflüssigkeit durch Autoradiographie.

16. Oligonukleotid der Formel X 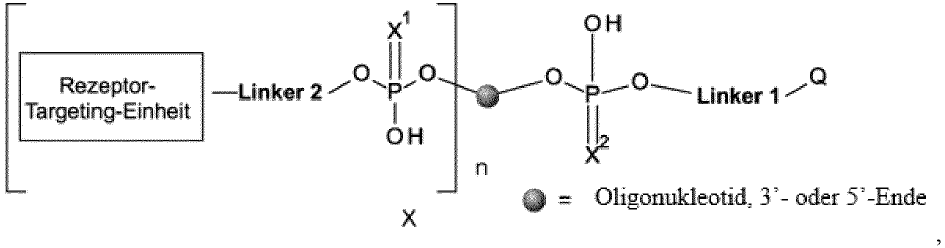 wobei
n 0 oder 1 ist;
X¹ und X² unabhängig voneinander S oder O sind;
Linker 1 eine C₂₋₁₂-Alkylenbrücke, eine Ethylenglykolbrücke, enthaltend 1 bis 10 Ethylenglykoleinheiten, oder eine Glycerol-basierte Brücke der Formel 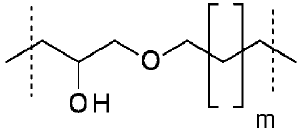
ist, wobei m eine ganze Zahl von 1 bis 6 ist;
Linker 2 eine gegebenenfalls aminogruppengeschützte Amino-C₂₋₁₂-alkylenbrücke, eine Aminoethylenglykolbrücke, enthaltend 1 bis 10 Ethylenglykoleinheiten, ist;
Q für einen Rest der Formel 2b' steht 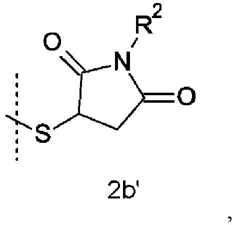
wobei R² eine C₁₋₆-Alkylgruppe ist; und
die Rezeptor-Targeting-Einheit eine Einheit ist, die dem Oligonukleotid zusätzliche Funktionalität verleiht.

17. Oligonukleotid nach Anspruch 16, wobei R² eine C₁₋₄-Alkylgruppe, vorzugsweise eine Methyl- oder Ethylgruppe ist.

18. Oligonukleotid nach einem der Ansprüche 16 oder 17, wobei das Oligonukleotid eine zusammenhängende Nukleotidsequenz aus 7 bis 30 Nukleotiden, bestehend aus gegebenenfalls modifizierten DNA-, RNA- oder LNA-Nukleosidmonomeren oder Kombinationen davon, umfasst.

19. Oligonukleotid nach Anspruch 16 der Formel Xb 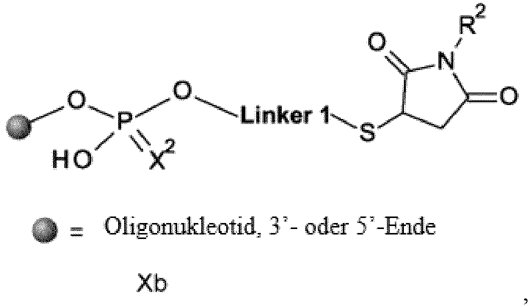 wobei R², X² und Linker 1 wie in Anspruch 16 definiert sind.

20. Oligonukleotid nach Anspruch 16 der Formel Xc 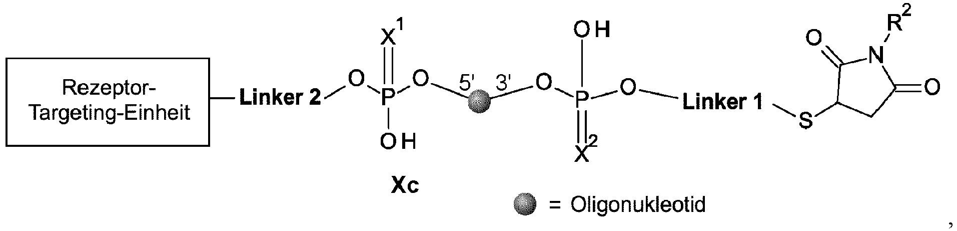 wobei R², X¹ und X², Linker 1 und Linker 2 wie in Anspruch 16 definiert sind.

21. Oligonukleotid nach Anspruch 16, wobei die Rezeptor-Targeting-Einheit eine Nicht-Nukleotideinheit, vorzugsweise eine Asialoglykoproteinrezeptor-Targeting-Einheit, stärker bevorzugt eine GalNAc-Einheit der Formel VII 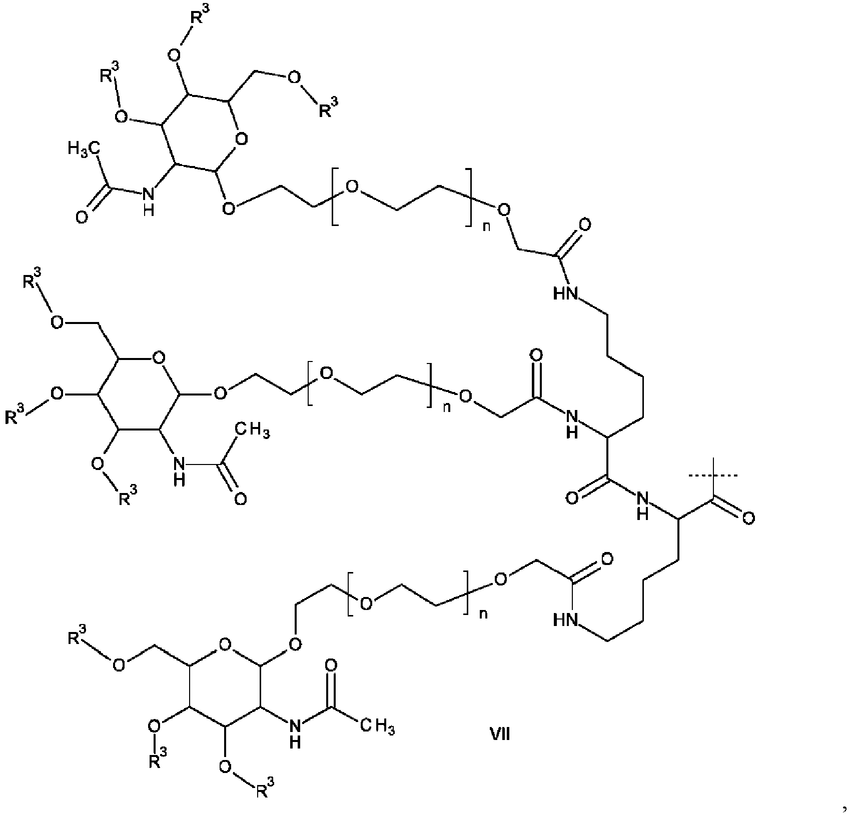 wobei R³ Wasserstoff oder eine Hydroxyschutzgruppe ist und n eine ganze Zahl von 0 bis 10, vorzugsweise von 0 bis 5, stärker bevorzugt von 1 bis 3, am stärksten bevorzugt aber 2 ist, entsprechende Salze, Enantiomere und/oder ein Stereoisomer davon ist.

## Revendications

1. Oligonucléotide radiomarqué de formule I 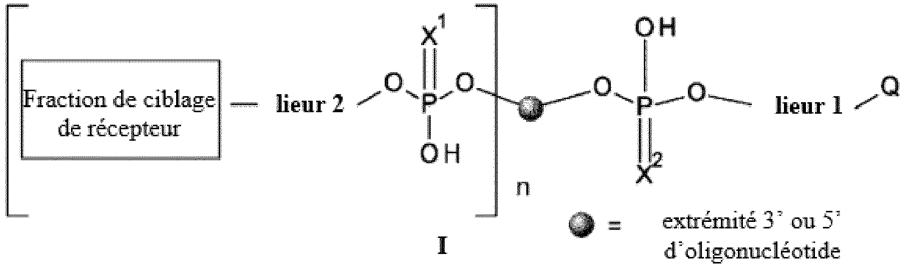 dans lequel,
n représente 0 ou 1 ;
X¹ et X² indépendamment l'un de l'autre représentent S ou O ;
lieur 1 représente un pont alkylène en C₂₋₁₂, un pont éthylène glycol contenant de 1 à 10 motifs éthylène glycol ou un pont à base de glycérol de formule 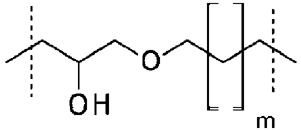
dans lequel m représente un nombre entier de 1 à 6 ;
lieur 2 représente un pont amino-alkylène en C₂₋₁₂ éventuellement protégé au niveau du groupe amino, un pont amino-éthylène glycol contenant de 1 à 10 motifs éthylène glycol ;
Q représente un résidu de formule 2a ou 2b 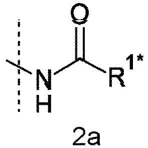 ou 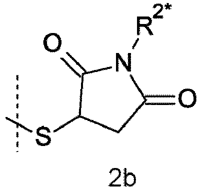
dans lequel R^{1*} et R^{2*} représentent un alkyle en C₁₋₆ marqué par ³H- ou ¹⁴C- ; et
la fraction de ciblage de récepteur est une fraction qui ajoute une fonctionnalité supplémentaire à l'oligonucléotide.

2. Oligonucléotide radiomarqué selon la revendication 1, dans lequel Q a la formule 2b et la conjugaison est au niveau de l'extrémité 3' ou 5' de l'oligonucléotide.

3. Oligonucléotide radiomarqué selon la revendication 1 ou 2, dans lequel Q a la formule 2a et la conjugaison est au niveau de l'extrémité 3' ou 5' de l'oligonucléotide.

4. Oligonucléotide radiomarqué selon l'une quelconque des revendications 1 à 3, dans lequel R^{1*} et R^{2*} représentent un alkyle en C₁₋₄ marqué par ³H- ou ¹⁴C, de préférence un méthyle ou un éthyle marqué par ³H- ou ¹⁴C.

5. Oligonucléotide radiomarqué selon l'une quelconque des revendications 1 à 4, dans lequel le marquage est avec ³H.

6. Oligonucléotide radiomarqué selon l'une quelconque des revendications 1 à 5, dans lequel l'oligonucléotide comprend une séquence nucléotidique contiguë de 7 à 30 nucléotides constitués de monomères nucléosidiques d'ADN, d'ARN ou de LNA éventuellement modifiés ou de combinaisons de ceux-ci.

7. Oligonucléotide radiomarqué selon la revendication 1 de formule Ib 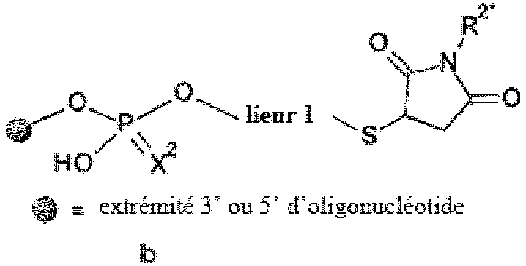 dans lequel R²*, X² et lieur 1 sont tels que définis dans la revendication 1.

8. Oligonucléotide radiomarqué selon la revendication 1 de formule Ic 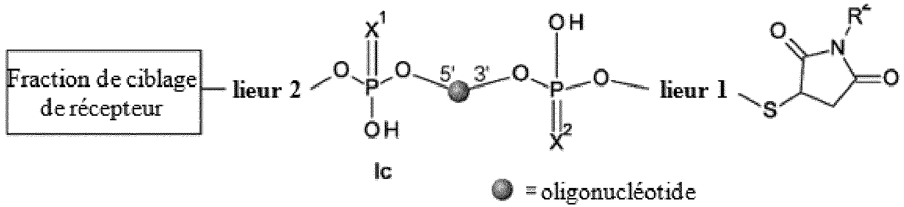 dans lequel R²*, X¹ et X², lieur 1 et lieur 2 sont tels que définis dans la revendication 1.

9. Oligonucléotide radiomarqué selon la revendication 1, dans lequel la fraction de ciblage de récepteur est une fraction non nucléotidique, de préférence une fraction de ciblage de récepteur de l'asialoglycoprotéine, plus préférablement une fraction GalNAc de formule VII 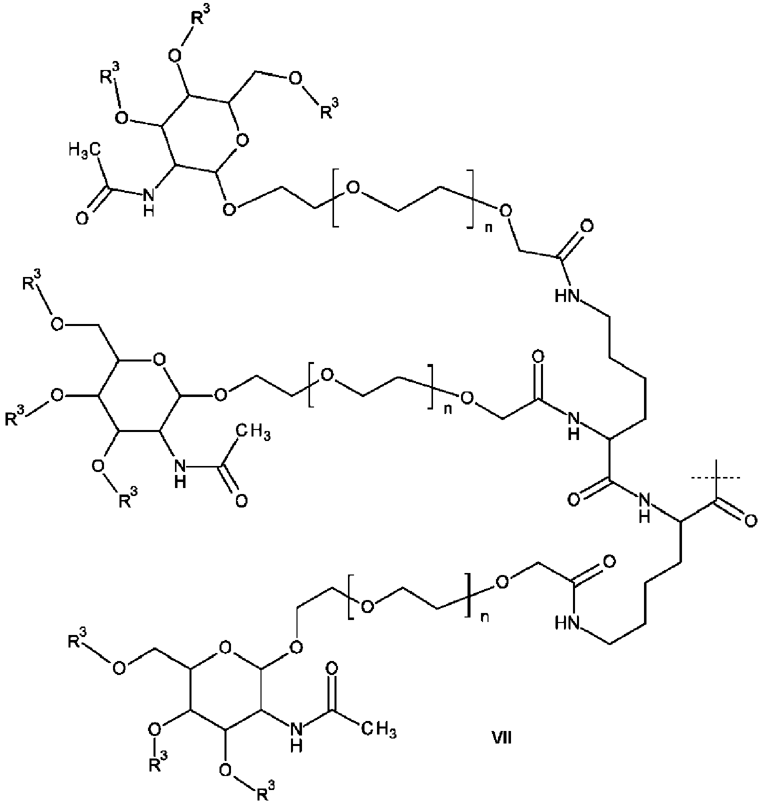 dans laquelle R³ représente un hydrogène ou un groupe protecteur d'hydroxy et n représente un nombre entier de 0 à 10, de préférence de 0 à 5, plus préférablement de 1 à 3, mais de manière préférée entre toutes n représente 2, des sels, des énantiomères et/ou un stéréoisomère correspondants de celle-ci.

10. Oligonucléotide radiomarqué selon la revendication 1 de formule Id 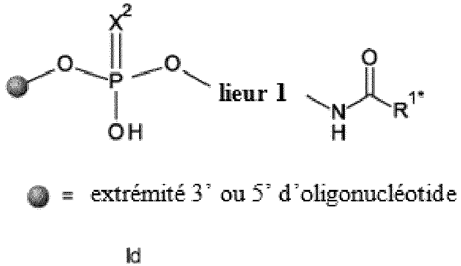 dans lequel R¹*, X² et lieur 1 sont tels que définis dans la revendication 1.

11. Oligonucléotide radiomarqué selon l'une quelconque des revendications 1 à 10 ayant une activité spécifique de 37 GBq/mmol (1 Ci/mmol) à 3,7 TBq/mmol (100 Ci/mmol), de préférence de 111 GBq/mmol (3 Ci/mmol) à 1,85 TBq/mmol (50 Ci/mmol), plus préférablement de 185 GBq/mmol (5 Ci/mmol) à 740 GBq/mmol (20 Ci/mmol).

12. Procédé de préparation d'un oligonucléotide radiomarqué de formule I selon la revendication 1, dans lequel Q représente le résidu de formule 2a comprenant la conjugaison d'une amine de formule III 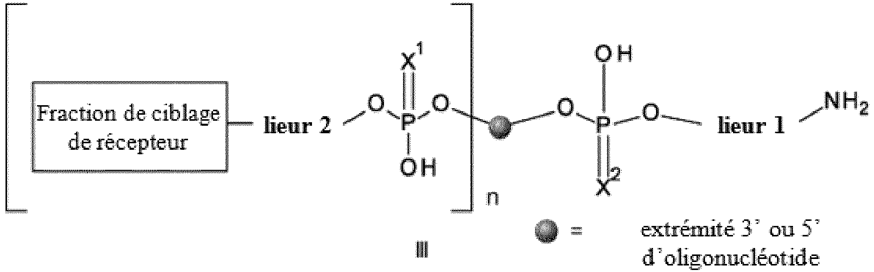 dans lequel,
n représente 0 ou 1 ;
X¹ et X² indépendamment l'un de l'autre représentent S ou O ;
lieur 1 représente un pont alkylène en C₂₋₁₂, un pont éthylène glycol contenant de 1 à 10 motifs éthylène glycol ou un pont à base de glycérol de formule 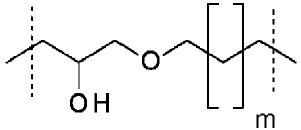
dans lequel m représente un nombre entier de 1 à 6 ;
lieur 2 représente un pont amino-alkylène en C₂₋₁₂ éventuellement protégé au niveau du groupe amino, un pont amino-éthylène glycol contenant de 1 à 10 motifs éthylène glycol ;
la fraction de ciblage de récepteur est une fraction non nucléotidique qui ajoute une fonctionnalité supplémentaire à l'oligonucléotide, en particulier une fraction de ciblage de récepteur de l'asialoglycoprotéine, de préférence une fraction GalNAc ;
avec un composé succinimide radiomarqué de formule IV 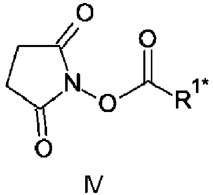
dans lequel R¹* est tel que défini dans la revendication 1.

13. Procédé de préparation d'un oligonucléotide radiomarqué de formule I selon la revendication 1, dans lequel Q représente le résidu de formule 2b comprenant la conjugaison d'un thiol de formule V 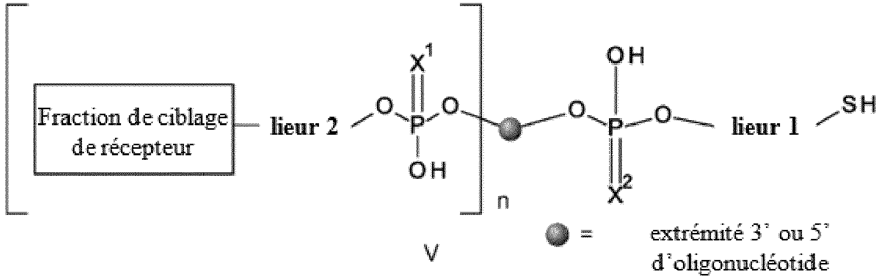 dans lequel,
n représente 0 ou 1 ;
X¹ et X² indépendamment l'un de l'autre représentent S ou O ;
lieur 1 représente un pont alkylène en C₂₋₁₂, un pont éthylène glycol contenant de 1 à 10 motifs éthylène glycol ou un pont à base de glycérol de formule 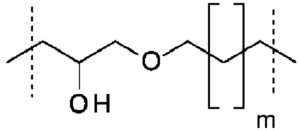
dans lequel m représente un nombre entier de 1 à 6 ;
lieur 2 représente un pont amino-alkylène en C₂₋₁₂ éventuellement protégé au niveau du groupe amino, un pont amino-éthylène glycol contenant de 1 à 10 motifs éthylène glycol ;
la fraction de ciblage de récepteur est une fraction non nucléotidique qui ajoute une fonctionnalité supplémentaire à l'oligonucléotide, en particulier une fraction de ciblage de récepteur de l'asialoglycoprotéine, de préférence une fraction GalNAc ;
avec un composé maléinimide radiomarqué de formule VI 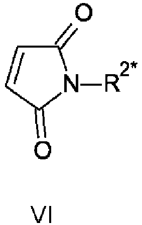
dans lequel R²* est tel que défini dans la revendication 1.

14. Utilisation de l'oligonucléotide radiomarqué selon l'une quelconque des revendications 1 à 11 pour la détermination de la biodistribution et de la pharmacocinétique de l'oligonucléotide dans un tissu ou un liquide corporel.

15. Procédé de détermination de la biodistribution et de la pharmacocinétique d'un oligonucléotide dans un tissu ou un liquide corporel comprenant :
a) l'administration d'une quantité efficace d'un oligonucléotide radiomarqué selon l'une quelconque des revendications 1 à 11 dans un tissu ou le liquide corporel à examiner et
b) la mesure de la biodistribution et de la pharmacocinétique de l'oligonucléotide radiomarqué selon l'une quelconque des revendications 1 à 11 dans un tissu ou liquide corporel et éventuellement
c) l'imagerie de l'oligonucléotide radiomarqué selon l'une quelconque des revendications 1 à 11 dans un tissu ou le liquide corporel à examiner par autoradiographie.

16. Oligonucléotide de formule X 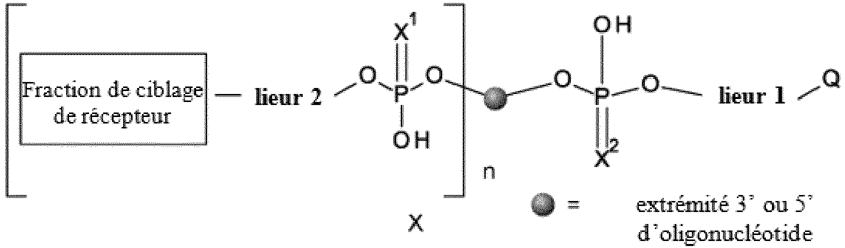 dans lequel,
n représente 0 ou 1 ;
X¹ et X² indépendamment l'un de l'autre représentent S ou O ;
lieur 1 représente un pont alkylène en C₂₋₁₂, un pont éthylène glycol contenant de 1 à 10 motifs éthylène glycol ou un pont à base de glycérol de formule 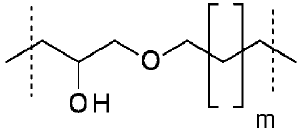
dans lequel m représente un nombre entier de 1 à 6 ;
lieur 2 représente un pont amino-alkylène en C₂₋₁₂ éventuellement protégé au niveau du groupe amino, un pont amino-éthylène glycol contenant de 1 à 10 motifs éthylène glycol ;
Q représente un résidu de formule 2b' 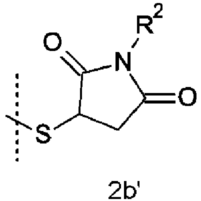
dans lequel R² représente un groupe alkyle en C₁₋₆ ; et
la fraction de ciblage de récepteur est une fraction qui ajoute une fonctionnalité supplémentaire à l'oligonucléotide.

17. Oligonucléotide selon la revendication 16, dans lequel R² représente un groupe alkyle en C₁₋₄, de préférence un groupe méthyle ou éthyle.

18. Oligonucléotide selon l'une quelconque des revendications 16 ou 17, dans lequel l'oligonucléotide comprend une séquence nucléotidique contiguë de 7 à 30 nucléotides constitués de monomères nucléosidiques d'ADN, d'ARN ou de LNA éventuellement modifiés ou de combinaisons de ceux-ci.

19. Oligonucléotide selon la revendication 16 de formule Xb 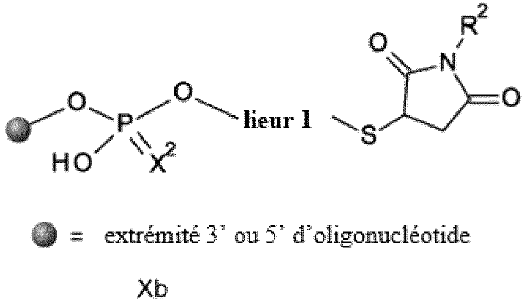 dans lequel R², X² et lieur 1 sont tels que définis dans la revendication 16.

20. Oligonucléotide selon la revendication 16 de formule Xc 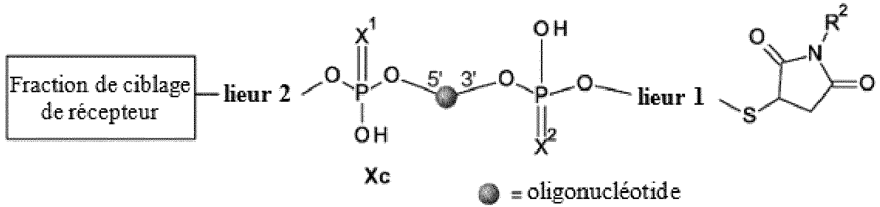 dans lequel R², X¹ et X², lieur 1 et lieur 2 sont tels que définis dans la revendication 16.

21. Oligonucléotide selon la revendication 16, dans lequel la fraction de ciblage de récepteur est une fraction non nucléotidique, de préférence une fraction de ciblage de récepteur de l'asialoglycoprotéine, plus préférablement une fraction GalNAc de formule VII 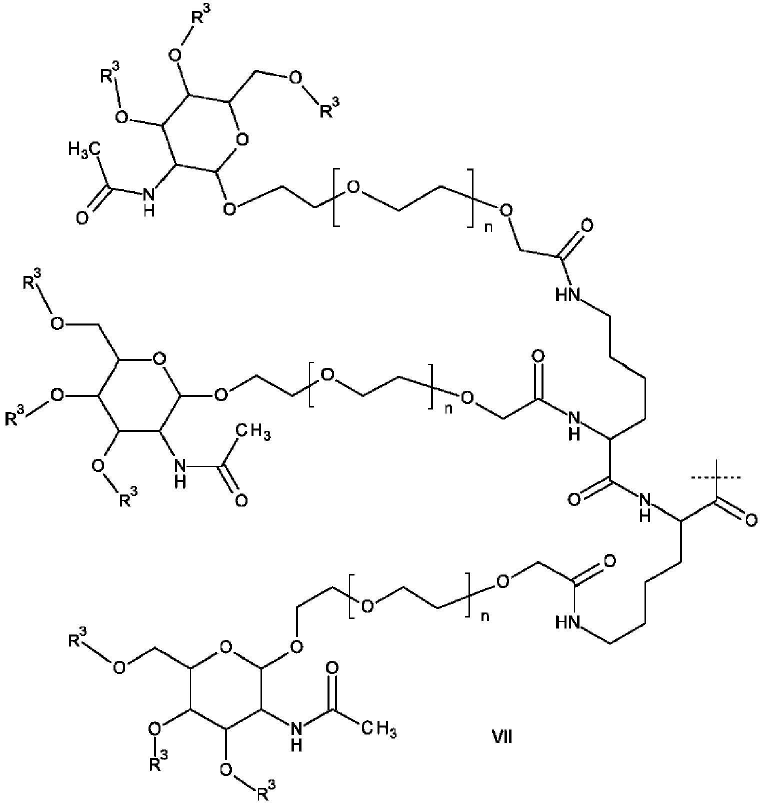 dans laquelle R³ représente un hydrogène ou un groupe protecteur d'hydroxy et n représente un nombre entier de 0 à 10, de préférence de 0 à 5, plus préférablement de 1 à 3, mais de manière préférée entre toutes n représente 2, des sels, des énantiomères et/ou un stéréoisomère correspondants de celle-ci.
